(19) Europäisches Patentamt
European Patent Office
Office européen des brevets

(11) EP 1 321 169 A1

(12) EUROPEAN PATENT APPLICATION

(43) Date of publication:
**25.06.2003 Bulletin 2003/26**

(21) Application number: **01130012.6**

(22) Date of filing: **18.12.2001**

(51) Int Cl.[7]: **A61P 29/00**, A61P 1/00, A61P 9/10, A61P 9/12, A61P 11/00, A61P 13/08, A61P 37/00, A61K 31/4172, A61K 45/06, A61K 31/505, A61K 31/445, A61K 31/415

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Biofrontera Pharmaceuticals AG**
**51377 Leverkusen (DE)**

(72) Inventors:
- **Engels, Peter, Dr.**
**51429 Bergisch Gladbach (DE)**
- **Ullmer, Christoph, Dr.**
**51467 Bergisch Gladbach (DE)**

(74) Representative:
**Sternagel, Fleischer, Godemeyer & Partner**
**Patentanwälte**
**Braunsberger Feld 29**
**51429 Bergisch Gladbach (DE)**

(54) **Combination of a serotonin receptor antagonist with a histidine decarboxylase inhibitor as a medicament**

(57) The present invention relates to the use of (S)-α-fluoromethylhistidine and esters and pharmaceutically acceptable salts thereof in combination with a serotonin receptor antagonist or a pharmaceutically acceptable salt thereof as a medicament and for the manufacture of a medicament for treatment of a disease state which can be alleviated by treatment with a serotonin receptor antagonist and/or a histamine receptor antagonist.

EP 1 321 169 A1

## Description

The present invention relates to a serotonin receptor antagonist in combination with a histidine decarboxylase inhibitor (HDC) and their pharmaceutically acceptable salts, which exhibit useful pharmacological properties, including utility as 5-$HT_{2B}$ receptor antagonists and a histidine decarboxylase inhibitor for treatment of a disease state which can be alleviated by treatment with a serotonin receptor antagonist and/or a histamine receptor antagonist.

Serotonin, a neurotransmitter with mixed and complex pharmacological characteristics, was first discovered in 1948, and subsequently has been the subject of substantial research. Serotonin, also referred to as 5-hydroxytryptamine (5-HT), acts both centrally and peripherally on discrete 5-HT receptors. Currently, fourteen subtypes of serotonin receptor are recognized and delineated into seven families, 5-$HT_1$, to 5-$HT_7$. Within the 5-$HT_2$ family, 5-$HT_{2A}$, 5-$HT_{2B}$ and 5-$HT_{2C}$ subtypes are known to exist. These subtypes share sequence homology and display similarities in their specificity for a wide range of ligands. Nomenclature and classification of 5-HT receptors have been reviewed recently (see Martin and Humphrey, Neuropharm. 1994, 33, 261-273 and Hoyer et al., Pharm. Rev. 1994, 46, 157-203).

The 5-$HT_{2B}$ receptor, initially termed 5-$HT_{2F}$, or serotonin-like receptor, was first characterized in rat isolated stomach fundus (see Clineschmidt et al., J. Pharmacol. Exp. Ther. 1985, 235, 696-708; Cohen and Wittenauer, J. Cardiovasc. Pharmacol. 1987, 10, 176-181) and initially cloned from rat (see Foguet et al., EMBO 1992, 11, 3481-3487) followed by the cloning of the human 5-$HT_{2B}$ receptor (see Schmuck et al., FEBS Lett. 1994, 342, 85-90; Kursar et al., Mol. Pharmacol. 1994, 46, 227-234). The 5-$HT_{2C}$ receptor, widely distributed in the human brain, was first characterized as a 5-$HT_{1C}$ subtype (see Pazos et al., Eur. J. Pharmacol. 1984, 106, 539-546) and was subsequently recognized as belonging to the 5-$HT_2$ receptor family (see Pritchett et al., EMBO J. 1988, 7, 4135-4.140).

Because of the similarities in the pharmacology of ligand interactions at 5-$HT_{2B}$ and 5-$HT_{2C}$ receptors, many of the therapeutic targets that have been proposed for 5-$HT_{2C}$ receptor antagonists are also targets for 5-$HT_{2B}$ receptor antagonists. Current evidence strongly supports a therapeutic role for 5-$HT_{2B/2C}$ receptor antagonists in treating anxiety (e.g., generalized anxiety disorder, panic disorder and obsessive compulsive disorder), alcoholism and addiction to other drugs of abuse, depression, migraine, sleep disorders, feeding disorders (e.g., anorexia nervosa) and priapism. Additionally, current evidence strongly supports a therapeutic role for selective 5-$HT_{2B}$ receptor antagonists that will offer distinct therapeutic advantages collectively in efficacy, rapidity of onset and absence of side effects. Such agents are expected to be useful in the treatment of hypertension, disorders of the gastrointestinal tract (e.g., irritable bowel syndrome, hypertonic lower esophageal sphinter, motility disorders), restenosis, asthma and obstructive airway disease, and prostate hyperplasia (e.g., benign prostate hyperplasia).

US-A-3,275,640 describes generically substituted 1-hydrocarbyl-4-(9H-thioxanthene-9-ylidene)-piperidines and their preparation. It is also disclosed that the compounds may be used as therapeutic agents because of their antihistaminic and/or antiserotonin properties.

US-A-3,557,287 relates to a combination preparation for use in the treatment of headaches of vascular origin containing as active constituents (a) a vasotonic lysergic acid selected from ergostine, ergotamine, dihydroergostine, dihydroergotamine, ergovaline, 5'-methylergoalanine; (b) caffeine; and (c) 9-(1-methyl-4-perperidylidene)thioxanthene (= 1-methyl-4-(9H-thioxanthene-9-ylidene)-piperidine.

DE-A-22 56 392 discloses 4-(9H-thioxanthene-9-ylidene)-piperidine derivatives wherein the nitrogen atom of the piperidine ring is bonded to an alkyl radical substituted with cyano, -COR or -COOR. Sleep-inducing properties are attributed to these derivatives.

JP-A-61106573 refers to the use of substituted 4-(9H-thioxanthene-9-ylidene)-piperidines as pesticides.

US-A 5,863,924 describes substances of the pyrimidine type for the use as selective 5$HT_{2B}$ receptor antagonists.

Numerous aryl substituted pyrimidine compounds have been exemplified in the chemical and patent literature. For example, Budesinsky et al., Collection Czechoslav. Chem. Commun., 26, 2865-2870 (1961), disclose 2-amino-6-methyl-4-(naphth-1-yl)-pyrimidine as an intermediate useful in the preparation of antibacterial compounds. Other pyrimidine derivatives are described in Mariella et al., J. Org. Chem., 25, 647-648 (1960); Zagulyaeva et al., Izv. Sib. Otd. Akad. Nauk SSSR, Ser. Khim. Nauk, 4, 27-31 (1990); Essawy et al., Egypt. J. Chem., 37(4), 423-31 (1994); U.S. Pat. Nos. 4,543,248, 4,619,933, 4,665,077, and 5,002,951, all to Stringfellow et al.; U.S. Pat. No. 5,147,876, Mizuchi et al.; U.S. Pat. No. 5,223,505, Hargreaves et al.; and European Patent Published Application EP 0 459 830, assigned to the Wellcome Foundation

Histamine plays an important role in allergy and inflammation. (S)-$\alpha$-fluoromethylhistidine (FMH) is an analogue of histidine and known to be a specific and potent inhibitor of the histidine decarboxylase (HDC), which forms histamine from histidine Watanabe et al. 1990, TIPS 11,363-367; Kollonitsch et al. 1978, Nature 278, 906-908). It acts selectively and irreversibly as a suicide substrate by formation of a covalent linkage, possibly with the serine residue in the active site of the enzyme (Bhattacharjee and Snell 1990, J. Biol. Chem.265, 6664-6668). Several studies revealed that inhibition of this enzyme could be useful in the treatment of patients with allergic diseases (Granus et al. 1985, Agents Action 16, 244-248; Pipkorn et al. 1987, Allergy 42, 496-501; Neittaamaki et al. 1989, Arch. Dermatol. Res.

281, 99-104).

British patent specification GB 2,001,626-A discloses α-halomethyl derivatives of amino acids, including α-fluoromethylhistidine for use as inhibitors of the activity of histidine decarboxylase.

EP 0 481 675 discloses the use of α-fluoromethylhistidine for the preparation of a medicament for treating asthma. In this application α-fluoromethylhistidine is not combined with a compound, which is able to affect 5-$HT_{2B}$ or 5-$HT_{2B}$receptors/HDC enzymes, therefore acting as a selective 5-$HT_{2B}$ or HDC inhibitor or 5-$HT_{2B}$ receptor antagonist/HDC inhibitor.

Object of the present invention is to provide a medicament for the treatment of a disease state which can be alleviated by treatment with a serotonin receptor antagonist and/or a histamine receptor antagonist which has long term effect with less sedative effects.

This object is met by a pharmaceutical composition containing a serotonin receptor antagonist and a histidine decarboxylase inhibitor or their pharmaceutically acceptable salts.

The present invention is also directed to the use of a histidine decarboxylase inhibitor and esters and pharmaceutically acceptable salts thereof in combination with a serotonin receptor antagonist or a pharmaceutically acceptable salt thereof for the manufacture of a medicament for the treatment of a disease state which can be alleviated by treatment with a serotonin receptor antagonist and/or a histamine receptor antagonist. Particularly a disease state which can be alleviated by treatment with a 5-$HT_{2B}$ receptor antagonist and/or a disease state whereby a HDC inhibitor substitutes the effect of a histamine receptor antagonist can be treated.

The present invention further relates to a pharmaceutical composition comprising a serotonin receptor antagonist and pharmaceutically acceptable salts thereof in combination with a histidine decarboxylase inhibitor or an ester or a pharmaceutically acceptable salt thereof, in admixture with one or more pharmaceutically acceptable carriers.

In one preferred embodiment of the present invention the serotonin receptor antagonist is a compound of the general formula

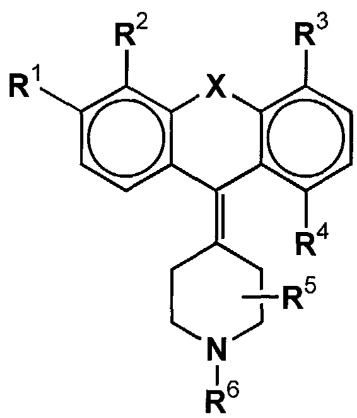

wherein R1 is methyl, ethyl, propyl, isopropyl, butyl, isobutyl, pentyl (straight or branched), hexyl (straight or branched), methoxy, ethoxy, propoxy, isopropoxy, butoxy, isobutoxy, phenoxy, trifluoromethyl, trifluoromethoxy, amino, dimethylamino, fluorine, chlorine, bromine, $-CON(CH_3)_2$ or $-CON(C_2H_5)_2$, R2 is methyl, ethyl, propyl , isopropyl, butyl, isobutyl, pentyl, hexyl, hydroxy or hydrogen, or R1 and R2 are forming a heterocycle, R3 is methyl, ethyl, propyl, isopropyl, butyl isobutyl, pentyl, hexyl, hydroxy or hydrogen, R4 is hydroxy, methoxy, ethoxy, propoxy, isopropoxy, butoxy, isobutoxy, trifluormethyl, amino, dimethylamino, diethylamino, fluorine, chlorine or bromine, methyl, ethyl, propyl, isopropyl, butyl or hydrogen, R5 is methyl or hydrogen, R6 is methyl or ethyl and X is S, N or Se, or a pharmaceutically acceptable salt thereof.

"Pharmaceutically acceptable salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

In a more preferred embodiment of the present invention the residues in the general formula shown above are as follows: R1 is an isopropyl, dimethylamino, methoxy or ethoxy group, R2 is methyl, ethyl or hydrogen, or R1 and R2 are forming a heterocycle, R3 is ethyl or hydrogen, R4 is methoxy, hydroxy, ethyl, methyl or hydrogen, R5 is methyl or hydrogen, R6 is methyl and X is S, N or Se, or a pharmaceutically acceptable salt thereof.

In a particularly preferred embodiment of the present invention the compound is selected of the group consisting of 1-methyl-4-(3-ethoxy-9H-thioxanthene-9-ylidene)-piperidine, 4-(6-Isopropyl-1-methoxy-thioxanthen-9-yliden)-1-methyl-piperidine, 4-(6-Isopropyl-1-hydroxy-thioxanthen-9-yliden)-1-methyl-piperidine, 4-(6-Ethoxy-1-ethyl-thioxanthen-9-yliden)-1-methyl-piperidine, 4-(6-Ethoxy-1-methoxythioxanthen-9-yliden)-1-methyl-piperidine, 4-(6-Ethoxy-1-hydroxy-thioxanthen-9-yliden)-1-methyl-piperidine, 4-(6-Methoxy-1-methoxy-thioxanthen-9-yliden)-1-methyl-piperidine, 4-(6-Dimethylamino-1-methoxy-thioxanthen-9-yliden)-1-methyl-piperidine, 4-(6-Dimethylamino-1-hydroxy-thioxanthen-9-yliden)-1-methyl-piperidine, 4-(3-Ethoxy-selenoxanthen-9-yliden)-1-methyl-piperidine, 4-(3-Ethoxy-1-methoxy-selenoxanthen-9-yliden)-1-methyl-piperidine, 4-(3-Ethoxy-1-hydroxy-selenoxanthen-9-yliden)-1-methyl-piperidine, 3-Ethoxy-9-(1-methyl-piperidine-4-ylidene)-9,10-dihydro-acridine, 6-Ethoxy-1-methoxy-9-(1-methyl-piperidine-4-ylidene)-9,10-dihydro-acridine, 6-Ethoxy-1-hydroxy-9-(1-methyl-piperidine-4-ylidene)-9,10-dihydro-acridine, 4-(3-Ethoxy-5-ethyl-thioxanthen-9-yliden)-1-methyl-piperidine, 4-(3-Ethoxy-4-methyl-thioxanthen-9-yliden)-1-methyl-piperidine, 4-(3-Ethoxy-4-ethyl-thioxanthen-9-yliden)-1-methyl-piperidine, 4-(3-Ethoxy-thioxanthen-9-yliden)-1,3-dimethyl-piperidine, 4-(3,4-(Cyclopent-3'-oxy-1'-eno)-thioxanthen-9-yliden)-1-methyl-piperidine, 4-(3,4-(Cyclopent-4'-oxy-1'-eno)-thioxanthen-9-yliden)-1-methyl-piperidine and 4-(3,4-(Cyclopent-5'-oxy-1'-eno)-thioxanthen-9-yliden)-1-methyl-piperidine.

In another preferred embodiment of the present invention the serotonin antagonist is a compound of the general formula

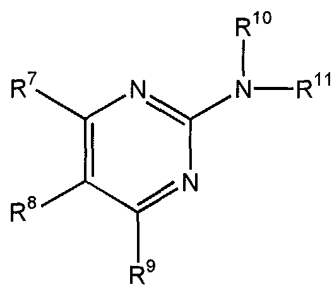

wherein $R^7$ is hydrogen, alkyl, hydroxyalkyl, cycloalkyl, cycloalkyl lower alkyl, alkenyl, lower thioalkoxy, halo, fluoroalkyl, $\text{--}NR^{12}$ $R^{13}$, $\text{--}CO_2R^{14}$, $\text{--}O(CH_2)_n$ $R^{15}$, or alkyl substituted with a substituent selected from the group consisting of hydroxy, alkoxy, halo, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl isoquinoline, isoquinolin-1-yl, isoquinolin-4-yl, isoqulnolin-8-yl, 3,4-dihydro-2H-quinolin-1-yl-1, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydro-quinoline, 1,2,3,4-tetrahydro-quinolin-1-yl, 1,2,3,4-tetrahydroquinolin-5-yl, indole, indol-1-yl, 1H-indol-4-yl, 1H-indol-7-yl, alkylylindol-3-yl, alkyl-1-H-indol-4-yl, 2,3-dihydroindole, 2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H,3H-benzo[de]isochromene, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, and benzo-1,4-dioxane;

in which

n is 1, 2, or 3;

$R^{12}$ and $R^{13}$, are hydrogen or lower alkyl;

$R^{14}$ is hydrogen or lower alkyl; and

$R^{15}$ is hydrogen, lower alkyl, hydroxy, hydroxy lower alkyl, lower alkylenyl, or lower alkoxy;

$R^8$ hydrogen, lower alkyl, lower alkoxy, halo, or lower fluoroalkyl;

$R^9$ is phenyl, alkylyphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl alkoxynaphth-2-yl, halonaphth-2-yl, acenaphthene, acenaphthen-5-yl, acenaphthen-6-yl, alkylacenaphthen-5-yl, anthracene, phenanthrene, phenanthrene-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydroquinoline, 1,2,3,4-tetrahydroquinolin-1-yl, 1,2,3,4-tetrahydroquinolin-5-yl, indole, indol-1-yl, 1H-indol-4-yl, 1H-indol-7-yl, alkylindol-3-yl, alkyl-1H-indol-4-yl, 2,3-dihydroindole, 2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H, 3H-benzo[de]isochromone, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, or benzo-1,4-dioxane;
$R^{10}$ is hydrogen, lower alkyl, cycloalkyl, alkylenyl, acyl, amino, amido, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydroquinoline, 1,2,3,4-tetrahydroquinolin-1-yl, 1,2,3,4-tetrahydro-quinolin-5-yl, indole, indol-1-yl, 1H-indol-4-yl, 1H-indol-7-yl, alkylindol-3-yl, alkyl-1H-indol-4-yl, 2,3-dihydroindole-2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo][1,4]oxazin-1-yl, 3,4-dihydro-2H-benzo[1,4]oxazin-5-yl, 1H,3H-benzo[de]isochromene, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, benzo-1,4-dioxane, $--(CH_2)_mNR^{16}R^{17}$, or lower alkyl substituted with a substituent selected from the group consisting of amino, monosubstituted amino, disubstituted amino, hydroxy, carboxy, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydro-quinoline, 1,2,3,4,-tetrahydroquinolin-1-yl, 1,2,3,4-tetrahydroquinolin-5-yl, indole, indol-1-yl, 1H-indol-4-yl, 1H-indol-7-yl, alkylindol-3-yl, alkyl-1H-indol-4-yl, 2,3-dihydroindole, 2,3-dihydroindol-1-yl, alkyl-2,3-dihydroindol-1-yl, 1-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H-3H-benzo[de]isochromene, 1H, 3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, benzo-1,4-dioxane, lower alkoxy, amido, alkoxy carbonyl, tetrahydrofuran-2-yl, hydroxyalkoxy, and sulfonamido;
in which
$R^{16}$ and $R^{17}$ are hydrogen or lower alkyl; and
$R^{11}$ is hydrogen or lower alkyl; provided that:

(i) when $R^9$ is naphthyl, indol-1-yl, or 2,3-dihydroindol-1-yl, and $R^8$, $R^{10}$ and $R^{11}$ are all hydrogen, $R^7$ is not hydrogen, methyl or ethyl;

(ii) when $R^9$ is phenyl or naphthyl, $R^7$ is not $-NR^{12}R^{13}$ ;

(iii) when $R^9$ is phenyl, $R^8$ is not lower alkoxy, and $R^7$ and $R^8$ are not halo;

(iv) when $R^6$ is phenyl and $R^7$ is H, $R^8$ is not hydrogen, methyl or ethyl; and

(v) when $R^9$ is 1,2,3,4-tetrahydroquinolinyl, $R^{10}$ and $R^{11}$ are hydrogen;

or a pharmaceutically acceptable salt or N-oxide thereof.
In a further particularly preferred embodiment the serotonin receptor antagonist is selected from the compounds of the following list:

2-amino-4-(2-methylnaphth-1-yl)-6-methylpyrimidine,
2-amino-4-(4-fluoronaphth-1-yl)-6-isopropylpyrimidine,

2-amino-4-(4-fluoronaphth-1-yl)-6-isopropylpyrimidine-1-N-oxide,
2-amino-4-(4-fluoronaphth-1-yl)-6-(2-methylpropyl)-pyrimidine,
2-amino-6-(tert-butyl)-4-(4-fluoronaphth-1-yl)-pyrimidine,
2-amino-4-(1H-indol-4-yl)-6-methylpyrimidine,
2-amino-4-(4-fluoronaphth-1-yl)-6-(1-fluoro-1-methylethyl)pyrimidine,
2-amino-4-(4-fluoronaphth-1-yl)-6-(1-hydroxy-1-methylethyl)pyrimidine,
4-(acenaphthene-5-yl)-2-amino-6-isopropylpyrimidine,
4-(acenaphthene-5-yl)-2-amino-6-tert-butylpyrimidine,
2-amino-6-ethyl-4-hydroxypyrimidine;
2-amino-4-hydroxy-6-isopropylpyrimidine,
2-amino-6-cyclobutyl-4-hydroxypyrimidine,
2-amino-6-cyclopentyl-4-hydroxypyrimidine,
2-amino-6-(but-2-yl)-4-hydroxypyrimidine,
2-amino-5,6-dimethyl-4-hydroxypyrimidine;
2-amino-6-benzyl-4-hydroxypyrimidine;
2-amino-6-cyclopropyl-4-hydroxypyrimidine;
2-amino-5-fluoro-4-hydroxy-6-methylpyrimidine;
2,6-diamino-4-hydroxypyrimidine;
2-amino-4-hydroxy-6-trifluoromethylpyrimidine; and
2-amino-4-hydroxy-6-phenylpyrimidine,
2-amino-4-chloro-6-tert-butylpyrimidine,
2-amino-4-chloro-6-ethylprimidine;
2-amino-4-chloro-6-isopropylpyrimidine,
2-amino-4-chloro-6-cyclopropylmethylpyrimidine,
2-amino-4-chloro-6-cyclobutylpyrimidine,
2-amino-6-(but-2-yl)-4-chloropyrimidine,
2-amino-4-chloro-6-cyclopentylpyrimidine,
2-amino-4-chloro-5,6-dimethylpyrimidine;
2-amino-6-benzyl-4-chloropyrimidine;
2-amino-4-chloro-6-cyclopropylpyrimidine;
2-amino-4-chloro-5-fluoro-6-methylpyrimidine;
2,6-diamino-4-chloropyrimidine;
2-amino-4-chloro-6-trifluoromethylpyrimidine,
2-amino-4-chloro-6-phenylpyrimidine.
4-hydroxy-6-methyl-2-(methylthio)pyrimidine,
4-hydroxy-6-isopropyl-2-(methylthio)pyrimidine;
6-tert-butyl-4-hydroxy-2-(methylthio)pyrimidine,
4-chloro-6-methyl-2-(methylthio)pyrimidine,
4-chloro-6-isopropyl-2-(methylthio)pyrimidine,
6-tert-butyl-4-chloro-2-(methylthio)pyrimidine,
4-(4-fluoronaphth-1-yl)-6-isopropyl-2-(methylthio)pyrimidine,
4-(4-fluoronaphth-1-yl)-6-methyl-2-(methylthio)pyrimidine,
4-(4-fluoronaphth-1-yl)-6-methoxy-2-(methylthio)pyrimidine,
4-(4-fluoronaphth-1-yl)-2-methylthio-6-phenethylpyrimidine,
4-(4-fluoronaphth-1-yl)-6-(2-hydroxyphenethyl)-2-(methylthio)pyrimidine;
4-(4-fluoronaphth-1-yl)-6-(3-hydroxypropyl)-2-(methylthio)pyrimidine,
4-(4-fluoronaphth-1-yl)-2-methanesulfonyl-6-phenethylpyrimidine,
4-(4-fluoronaphth-1-yl)-6-(2-hydroxyphenethyl)-2-methanesulfonylpyrimidine,
4-(4-fluoronaphth-1-yl)-6-(3-hydroxypropyl)-2-methanesulfonylpyrimidine,
4-(4-fluoronaphth-1-yl)-6-methoxy-2-methanesulfonylpyrimidine,
4-(4-fluoronaphth-1-yl)-6-isopropyl-2-methanesulfonylpyrimidine
2-amino-6-cyclopentyl-4-(naphth-1-yl)-pyrimidine,
2-amino-6-(but-2-yl)-4-(naphth-1-yl)-pyrimidine,
2-amino-6-(2-methylpropyl)-4-(naphth-1-yl)-pyrimidine hydrobromide,
2-amino-6-(tert-butyl)-4-(naphth-1-yl)-pyrimidine,
2-amino-6-benzyl-4-(naphth-1-yl)-pyrimidine,
2-amino-6-cyclobutyl-4-(naphth-1-yl)-pyrimidine,
2-amino-6-cyclopropyl-4-(naphth-1-yl)-pyrimidine,

2-amino-4-(naphth-1-yl)-6-n-propylpyrimidine,
2-amino-6-isopropyl-4-(naphth-1-yl)-pyrimidine,
2-amino-5-fluoro-6-methyl-4-(naphth-1-yl)-pyrimidine,
2-amino-6-ethyl-4-(naphth-1-yl)-pyrimidine hydrochloride,
2,6-diamino-4-(naphth-1-yl)-pyrimidine hydrochloride,
2-amino-6-trifluoromethyl-4-(naphth-1-yl)-pyrimidine,
2-amino-4-(naphth-1-yl)-6-phenylpyrimidine hydrochloride,
2-amino-4-(3-fluorophenyl)-6-methylpyrimidine,
2-amino-4-(5-chlorothiophen-2-yl)-6-methylpyrimidine,
2-amino-4-(3-methoxyphenyl)-6-methylpyrimidine,
2-amino-6-methyl-4-(3-n-triphenyl)-pyrimidine,
2-amino-4-(3-chloro-4-fluorophenyl)-6-methylpyrimidine,
2-amino-4-(3,5-dichlorophenyl)-6-methylpyrimidine,
2-amino-6-methyl-4-(3-trifluoromethylphenyl)-pyrimidine,
2-amino-6-methyl-4-(naphth-1-yl)-pyrimidine hydrochloride,
2-amino-4-(4-amino-5-chloro-2-methoxyphenyl)-6-isopropylpyrimidine hydrochloride,
2-amino-6-(3-methylbutyl)-4-(naphth-1-yl)-pyrimidine hydrochloride
2-amino-4-(4-amino-5-chloro-2-methoxyphenyl)-6-methylpyrimidine,
2-amino-4-(4-chloronaphth-1-yl)-6-(2-methylpropyl)-pyrimidine hydrochloride,
2-amino-4-(4-fluoronaphth-1-yl)-6-(2-methylpropyl)-pyrimidine hydrochloride,
2-amino-4-(4-chloronaphth-1-yl)-6-ethylpyrimidine,
2-amino-4-(4-methylnaphth-1-yl)-6-isopropylpyrimidine,
2-amino-6-(tert-butyl)-4-(4-fluoronaphth-1-yl)-pyrimidine
2-amino-4-(4,5-dimethylnaphth-1-yl)-6-methylpyrimidine,
2-amino-4-(4,5-difluoronaphth-1-yl)-6-isopropylpyrimidine,
2-amino-4-(4-chloronaphth-1-yl)-6-isopropylpyrimidine hydrochloride,
2-amino-6-cyclopropyl-4-(4-fluoronaphth-1-yl)-pyrimidine,
2-amino-6-cyclopropylmethyl-4-(4-fluoronaphth-1-yl)-pyrimidine hydrochloride,
2-amino-6-cyclobutyl-4-(4-fluoronaphth-1-yl)-pyrimidine hydrochloride,
2-amino-4-(4,5-difluoronaphth-1-yl)-6-methylpyrimidine,
2-amino-4-(1H,3H-benzo[de]isochromen-6-yl)-6-methylpyrimidine,
4-(acenaphthen-5-yl)-2-amino-6-isopropylpyrimidine,
2-amino-6-methyl-4-(phenanthren-9-yl)-pyrimidine,
2-amino-4-(4-methylnaphth-1-yl)-6-methylpyrimidine,
2-amino-4-(4-fluoronaphth-1-yl)-6-isopropylpyrimidine hydrochloride,
2-amino-4-(4-fluoronaphth-1-yl)-6-isopropylpyrimidine maleate,
2-amino-6-ethyl-4-(2-methyl-4-fluoronaphth-1-yl)-pyrimidine,
4-(acenaphthen-5-yl)-2-amino-6-methylpyrimidine,
2-amino-4-(isoquinolin-4-yl)-6-methylpyrimidine,
2-amino-6-methyl-4-(quinolin-8-yl)-pyrimidine,
2-amino-4-(4-fluoronaphth-1-yl)-pyrimidine,
2-amino-6-ethyl-4-(4-fluoronaphth-1-yl)-pyrimidine hydrochloride,
2-amino-4-(4-fluoronaphth-1-yl)-6-methylpyrimidine hydrochloride,
2-amino-4-(2-methylnaphth-1-yl)-6-methylpyrimidine hydrochloride,
2-amino-4-(4-fluoronaphth-1-yl)-6-(3,3,3-trifluoropropyl)-pyrimidine hydrochloride,
2-amino-4-(5-fluoronaphth-1-yl)-6-isopropylpyrimidine,
2-amino-4-(2-fluoronaphth-1-yl)-6-isopropylpyrimidine hydrochloride,
2-amino-4-(2-fluoronaphth-1-yl)-6-methoxypyrimidine hydrochloride,
2-amino-4-(4-fluoronaphth-1-yl)-6-methoxypyrimidine hydrochloride,
2-amino-4-(4-fluoronaphth-1-yl)-6-(2,2,2-trifluoroethoxy)-pyrimidine hydrochloride,
2-amino-6-tert-butyl-4-(2-fluoronaphth-1-yl)-pyrimidine hydrochloride,
2-amino-4-(2-fluoronaphth-1-yl)-6-methylpyrimidine,
2-amino-6-isopropyl-4-(2-methylnaphth-1-yl)-pyrimidine hydrochloride,
2-amino-4-(6-methylacenaphthen-5-yl)-6-methylpyrimidine,
2-amino-6-cyclopropyl-4-(1H-indol-4-yl)-pyrimidine hydrochloride,
2-amino-6-tert-butyl-4-(1H-indol-4-yl)-pyrimidine,
2-amino-4-(8-hydroxymethylnaphth-1-yl)-6-methylpyrimidine,
2-amino-4-(1H-indol-7-yl)-6-isopropylpyrimidine,

2-amino-6-cyclobutyl-4-(1H-indol-4-yl)-pyrimidine,
4-(acenaphthen-5-yl)-2-amino-6-methoxypyrimidine hydrochloride,
4-(acenaphthen-5-yl)-2-amino-6-cyclopropylpyrimidine,
4-(acenaphthen-5-yl)-2-amino-6-tert-butylpyrimidine hydrochloride,
2-amino-4-(6-fluoro-3,4-dihydro-2H-quinolin-1-yl)-6-methylpyrimidine,
2-amino-6-chloro-4-(3,4-dihydro-2H-quinolin-1-yl)-pyrimidine hydrochloride,
2-amino-4-(indol-1-yl)-6-methylpyrimidine hydrochloride,
2,6-diamino-4-(3,4-dihydro-2H-quinolin-1-yl)-pyrimidine dihydrochloride, 6-(3,4-dihydro-2H-quinolin-1-yl)-9H-purin-2-ylamine,
2-amino-4-(2-methyl-3,4-dihydro-2H-quinolin-1-yl)-6-methylpyrimidine,
2-amino-4-(6-methoxy-3,4-dihydro-2H-quinolin-1-yl)-6-trifluoromethylpyrimidine,
2-amino-4-(3,4-dihydro-2H-quinolin-1-yl)-6-ethylpyrimidine,
2-amino-6-methyl-4-(6-methyl-3,4-dihydro-2H-quinolin-1-yl)-pyrimidine,
2-amino-4-(3,4-dihydro-2H-quinolin-1-yl)-6-trifluoromethylpyrimidine,
2-amino-4-(6-fluoro-2-methyl-3,4-dihydro-2H-quinolin-1-yl)-6-methylpyrimidine,
2-amino-6-methyl-4-(2,3,4,5-tetrahydro-benzo[b]azepin-1-yl)-pyrimidine,
2-amino-4-(7,8-dihydro-6H-5-oxa-9-azabenzocyclohepten-9-yl)-6-methylpyrimidine,
2-amino-4-(2,3-dihydro-benzo[1,4]oxazin-4-yl)-6-methylpyrimidine,
2-amino-4-(2,3-dihydro-indol-1-yl)-6-methylpyrimidine,
2-amino-4-(2-methyl-2,3-dihydro-indol-1-yl)-6-methylpyrimidine,
2-amino-4-(3,4-dihydro-2H-quinolin-1-yl)-6-methylpyrimidine hydrochloride,
2-amino-4-(3,4-dihydro-1H-isoquinolin-2-yl)-6-methylpyrimidine,
2-amino-4-(3,4-dihydro-2H-quinolin-1-yl)-6-isopropylpyrimidine hydrochloride,
2-amino-4-(6-fluoro-3,4-dihydro-2H-quinolin-1-yl)-6-isopropylpyrimidine hydrochloride,
2-amino-4-(3,4-dihydro-2H-quinolin-1-yl)-6-tert-butylpyrimidine hydrochloride,
2-amino-4-(1H-indol-4-yl)-6-methylpyrimidine
2-amino-5-(6-methyl-4-naphth-1-yl)-pyrimidin-2-ylamino)-pentanoic acid
2-amino-6-methyl-4-(naphth-1-yl)-pyrimidine hydrochloride
2-amino-4-(3-chlorophenyl)-6-methylpyrimidine
2-amino-4-(2,3-dihydro-1,4-benzodioxin-5-yl)-6-methylpyrimidine hydrochloride
2-amino-4-(1-methylindol-3-yl)-pyrimidine hydrochloride
2-amino-4-(4,7-difluoronaphth-1-yl)-6-isopropylpyrimidine
2-amino-4-(4,6-difluoronaphth-1-yl)-6-isopropylpyrimidine hydrochloride,
2-amino-4-(4,8-difluoronaphth-1-yl)-6-isopropylpyrimidine hydrochloride,
2-amino-4-(4-methoxynaphth-1-yl)-6-isopropylpyrimidine hydrochloride,
2-amino-6-tert-butyl-4-(4-methoxynaphth-1-yl)-pyrimidine hydrochloride,
2-amino-4-(1H-indol-4-yl)-6-isopropylpyrimidine hydrochloride,
2-amino-4-(1-methyl-1H-indol-4-yl)-6-isopropylpyrimidine,
2-amino-4-(4-fluoronaphth-1-yl)-6-(1-fluoro-1-methylethyl)-pyrimidine,
2-amino-4-(4-fluoronaphth-1-yl)-6-(1-fluoro-1-methylethyl)-pyrimidine hydrochloride,
4-(4-fluoronaphth-1-yl)-6-(1-fluoro-1-methylethyl)-2-methylaminopyrimidine,
2-amino-4-(4-methoxynaphth-1-yl)-6-methylpyrimidine hydrochloride,
2-amino-6-ethyl-4-(4-methoxynaphth-1-yl)-pyrimidine hydrochloride,
2-amino-4-(4,6-difluoronaphth-1-yl)-6-(1-fluoro-1-methylethyl)-pyrimidine hydrochloride,
2-amino-4-(acenaphthen-5-yl)-6-(1-methyl-1-fluoroethyl)-pyrimidine,
2-amino-6-chloro-4-(naphth-1-yl)-pyrimidine hydrochloride,
2-amino-4-(naphth-1-yl)-pyrimidine hydrochloride
2-amino-4-methoxy-6-(naphth-1-yl)-pyrimidine hydrochloride
2-amino-6-(2-hydroxyethoxy)-4-(naphth-1-yl)-pyrimidine hydrochloride,
2-amino-6-isopropyloxy-4-(naphth-1-yl)-pyrimidine hydrochloride,
2-amino-6-ethoxy-4-(naphth-1-yl)-pyrimidine hydrochloride,
2-amino-6-methylthio-4-(naphth-1-yl)-pyrimidine hydrochloride,
6-isopropylpyrimidine (4-(4-fluoronaphth-1-yl)-6-isopropylpyrimidine
4-(4-fluoronaphth-1-yl)-2-hydrazino-6-isopropylpyrimidine hydrochloride,
4-(4-fluoronaphth-1-yl)-6-isopropyl-2-(piperazin-1-yl)-pyrimidine fumarate,
4-(4-fluoronaphth-1-yl)-6-isopropyl-2-(2-methoxyethylamino)-pyrimidine,
4-(4-fluoronaphth-1-yl)-6-isopropyl-2-n-propylamino-pyrimidine,
2-allylamino-4-(4-fluoronaphth-1-yl)-6-isopropylpyrimidine,

4-(4-fluoronaphth-1-yl)-6-isopropyl-2-(piperidin-1-yl)-pyrimidine,
2-benzylamino-4-(4-fluoronaphth-1-yl)-6-isopropylpyrimidine,
2-cyclopropylamino-4-(4-fluoronaphth-1-yl)-6-isopropylpyrimidine,
2-(2-hydroxyethylamino)-4-(4-fluoronaphth-1-yl)-6-isopropylpyrimidine hydrochloride,
4-(4-fluoronaphth-1-yl)-6-isopropyl-2-morpholinopyrimidine,
2-butylamino-4-(4-fluoronaphth-1-yl)-6-isopropylpyrimidine,
2-butylamino-4-(4-fluoronaphth-1-yl)-6-methylpyrimidine hydrochloride,
2-dimethylamino-4-(4-fluoronaphth-1-yl)-6-isopropylpyrimidine,
4-(4-fluoronaphth-1-yl)-6-isopropyl-2-methylaminopyrimidine,
4-(4-fluoronaphth-1-yl)-6-(2-hydroxy-2-phenethyl)-2-methylaminopyrimidine,
4-(4-fluoronaphth-1-yl)-6-phenethyl-2-methylaminopyrimidine hydrochloride,
4-(4-fluoronaphth-1-yl)-2-isopropylamino-6-methoxypyrimidine hydrochloride,
2-(dimethylaminoethyl)amino-4-(4-fluoronaphth-1-yl)-6-isopropypyrimidine hydrochloride,
4-(4-fluoronaphth-1-yl)-6-isopropy-2-(methylaminoethyl)amino-pyrimidine hydrochloride,
4-(4-fluoronaphth-1-yl)-6-(2-hydroxypropyl)-2-(methylamino)ethylamino-pyrimidine hydrochloride,
2-(2-hydroxyethyl)amino-4-(4-fluoronaphth-1-yl)-6-methoxypyrimidine hydrochloride,
6-tert-butyl-4-(4-fluoronaphth-1-yl)-2-methylamino-pyrimidine,
2-benzylamino-6-tert-butyl-4-(4-fluoronaphth-1-yl)-pyrimidine,
6-tert-butyl-4-(4-fluoronaphth-1-yl)-2-isopropylamino-pyrimidine hydrobromide,
6-tert-butyl-4-(4-fluoronaphth-1-yl)-2-(2-methoxyethyl)amino-pyrimidine hydrochloride,
4-(4-fluoronaphth-1-yl)-6-isopropyl-2-(pyridin-4-yl)methylamino-pyrimidine,
2-(2-amino)ethylamino-4-(4-fluoronaphth-1-yl)-6-isopropyl-pyrimidine fumarate,
4-(4-fluoronaphth-1-yl)-6-isopropyl-2-(4-methoxyphenyl)methylamino-pynmidine hydrochloride,
4-(4-fluoronaphth-1-yl)-2-(tetrahydro-2-furyl)methylamino-6-isopropyl-pyrimidine,
4-(4-fluoronaphth-1-yl)-2-(2-hydroxyethyl)amino-6-isopropyl-pyrimidine maleate,
4-(4-fluoronaphth-1-yl)-2-(2-hydroxyethoxyethyl) amino-6-isopropyl-pyrimidine hydrobromide,
2-(1,3-dihydroxyprop-2-yl)amino-4-(4-fluoronaphth-1-yl)-6-isopropyl-pyrimidine maleate,
2-amino-4-(4-fluoronaphth-1-yl)-6-(2-methoxyethyl)pyrimidine maleate,
2-amino-4-(4-fluoronaphth-1-yl)-6-phenethylpyrimidine maleate,
2-(2-aminoethyl)amino-6-tert-butyl-4-(4-fluoronaphth-1-yl)-pyrimidine fumarate,
4-(4-fluoronaphth-1-yl)-6-isopropyl-2-phenylaminopyrimidine,
2-amino-6-methyl-4-(naphth-1-yl)-pyrimidine-1-N-oxide,
2-amino-4-(4-fluoronaphth-1-yl)-6-isopropylpyrimidine-3-N-oxide,
2-amino-6-tert-butyl-4-(4-fluoronaphth-1-yl)-pyrimidine-3-N-oxide,
2-amino-4-(4-fluoronaphth-1-yl)-6-isopropylpyrimidine-1-N-oxide hydrochloride,
2,6-diamino-4-(naphth-1-yl)-pyrimidine-1-N-oxide,
2-amino-4-(4-fluoronaphth-1-yl)-6-isopropylpyrimidine-1-N-oxide,
4-(4-fluoronaphth-1-yl)-6-isopropylpyrimidine-2-methylamino-1-N-oxide,
2-amino-4-(acenaphthen-5-yl)-6-isopropylpyrimidine-1-N-oxide,
2-amino-4-(4-fluoronaphth-1-yl)-6-(1-hydroxy-1-methylethyl)-pyrimidine,
4-(4-fluoronaphth-1-yl)-6-isopropenylpyrimidine,
2-acetylamino-4-(4-fluoronaphth-1-yl)-6-isopropylpyrimidine,
2-acetylamino-4-(4-fluoronaphth-1-yl)-6-isopropylpyrimidine-1-N-oxide,
2-(bis-methanesulfonyl)amino-4-(4-fluoronaphth-1-yl)-6-isopropylpyrimidine,
2-(methanesulfonyl)amino-4-(4-fluoronaphth-1-yl)-6-isopropylpyrimidine,
1-[4-(4-fluoronaphth-1-yl)-6-isopropyl-pyrimidin-2-yl]-3-phenylurea,
2-amino-5-bromo-4-(4-fluoronaphth-1-yl)-6-isopropyl-pyrimidine,
2-amino-4-(4-methylthionaphth-1-yl)-6-isopropylpyrimidine,
2-amino-4-(4-aminonaphth-1-yl)-6-isopropyl-pyrimidine,
2-(2-methanesulfonamidoethyl)amino-4-(4-fluoronaphth-1-yl)-6-isopropylpyrimidine,
6-tert-butyl-4-(4-fluoronaphth-1-yl)-2-(2-methanesulfonamidoethyl)amino-pyrimidine hydrochloride,
2-[6-isopropyl-4-(4-fluoronaphth-1-yl)pyrimidin-2-ylamino]-N-methyl-propionamide,
2-[6-isopropyl-4-acenaphthen-5-yl)pyrimidin-2-ylamino]-N-methyl-propionaimde,
2-(6-isopropyl-4-(4-fluoronaphth-1-yl)-pyrimidin-2-ylamino)-propionic acid methyl ester maleate,
2-(6-isopropyl-4-(4-fluoronaphth-1-yl)-pyrimidin-2-ylamino)-propionic acid,
2-(4-acenaphthen-5-yl-6-isopropyl-pyrimidin-2-ylamino)-propionic acid methyl ester,
2-amino-4-(4-fluoronaphth-1-yl)-6-isopropylpyrimidine,
4-(3,4-dihydro-2H-quinolin-1-yl)-2-(methylamino)-6-methylpyrimidine,

2-amino-6-chloro-4-(4-methoxyphenyl)-5-methylpyrimidine-1-N-oxide.

As used herein:

"Alkyl" means a branched or unbranched saturated hydrocarbon chain containing 1 to 12 carbon atoms, such as methyl, ethyl, propyl, isopropyl, butyl, tert-butyl, pentyl, n-hexyl, n-heptyl, n-octyl, n-decyl, n-dodecyl, and the like.

"Alkenyl" refers to an unsaturated monovalent hydrocarbon radical of 1 to 12 carbon atoms. This term is further exemplified by such radicals as vinyl, prop-2-enyl, pent-3-enyl, hex-5-enyl, oct-2-enyl, and the like.

"Cycloalkyl" means a monovalent saturated carbocyclic radical containing no unsaturation and having from three to eight carbon atoms, e.g., cyclopropyl, 2-methylcyclopropyl, cyclobutyl, 3-ethylcyclobutyl, cyclopentyl, cyclohexyl, cycloheptyl, cyclooctyl,

"Lower alkyl" means a branched or unbranched saturated hydrocarbon chain containing 1 to 6 Carbon atoms, such as methyl, ethyl, propyl, isopropyl, tert-butyl, butyl, pentyl, n-hexyl and the like, unless otherwise indicated.

"Lower alkenyl" refers to an unsaturated monovalent hydrocarbon radical of one to six carbon atoms. This term is further exemplified by such radicals as vinyl, prop-2-enyl, pent-3-enyl, and hex-5-enyl.

"Cycloalkyl lower alkyl" as defined herein means cycloalkyl as defined above attached to a lower alkyl radical as defined above, for example e.g., cyclopropylmethyl, cyclopropylethyl, cyclopropylpropyl, cyclobutylmethyl, cyclobutylethyl, cyclopentylmethyl, cyclohexylmethyl, cycloheptylmethyl, cyclooctylmethyl, and the like.

"Phenyl lower alkyl" means phenyl attached to a lower alkyl radical as defined above, for example phenylmethyl (benzyl), phenethyl, phenylpropyl, and the like.

"Fluoroalkyl" means alkyl as defined above substituted by 1 to 5 fluorine atoms in any position, for example trifluoromethyl, pentafluoroethyl, 1,1,1-trifluoro-n-propyl, 1-fluoro-n-butyl, 1,2-difluoro-3-methylpentane, 1-fluorooctane, and the like.

"Lower fluoroalkyl" means lower alkyl as defined above substituted by 1 to 5 fluorine atoms in any position, for example trifluoromethyl, pentafluoroethyl, 1,1,1-trifluoro-n-propyl, 1-fluoro-n-butyl, 1,2-difluoro-3-methylpentane, and the like.

"Acyl" refers to the group --C(O)--R', where R' is lower alkyl as herein defined.

"Lower alkoxy" means the group --0--R' wherein R' is lower alkyl as herein defined. Likewise, "lower thioalkoxy" denotes the group --S--R'.

"Hydroxyalkyl" means the group alkyl as defined above substituted by 1, 2 or 3 hydroxy groups, for example hydroxymethyl, 1-hydroxyethyl, 2-hydroxyethyl, 1,2-dihydroxyethyl, 1-hydroxyisopropyl, 2-hydroxyisopropyl, 1,2-dihydroxyisopropyl, 1-hydroxybutyl, 1,3-dihydroxybutyl, and the like. Similarly, "hydroxy lower alkyl" means the group lower alkyl as defined above substituted by 1, 2 or 3 hydroxy groups.

"Halo" denotes fluoro, chloro, bromo, or iodo, unless otherwise indicated.

"Monosubstituted amino" means a radical --NHR where R is lower alkyl or optionally substituted lower alkyl as herein defined, for example, methylamino, ethylamino, or the like.

"Disubstituted amino" means a radical --$NR^a R^b$, where $R^a$ and $R^b$ are independently selected from lower alkyl or optionally substituted lower alkyl as herein defined, for example dimethylamino, diethylamino.

"Alkoxycarbonyl" means a radical --C(O)$OR^c$ where $R^c$ is lower alkyl or aryl as herein defined, for example methoxycarbonyl or phenoxycarbonyl.

"Amido" means a radical --C(O)$NR^d R^c$ where $R^d$ and $R^c$ are independently hydrogen, lower alkyl, or aryl as herein defined.

"Sulfonamido" means a radical --$NR^fSO_2R^g$ where $R^f$ and $R^g$ are independently hydrogen or lower alkyl as herein defined, for example methanesulfonamido.

"Hydroxyalkoxy" means lower alkoxy as herein defined substituted with a hydroxy group, for example 2-hydroxyethoxy.

"Optional" or "optionally" means that the subsequently described event or circumstance may or may not occur, and that the description includes instances where said event or circumstance occurs and instances in which it does not. For example, "optionally substituted phenyl" or "optionally substituted aryl" means that phenyl or aryl may or may not be substituted with a substituent selected from the group consisting of lower alkyl, lower alkoxy, hydroxy, nitro, lower fluoroalkyl, and halo, and encompasses unsubstituted phenyl and unsubstituted aryl and all possible isomeric phenyl and aryl radicals that are mono, di or trisubstituted. Similarly, "lower alkyl optionally substituted by" means that lower alkyl may or may not be substituted with a substituent selected from the group consisting of amino, monosubstituted amino, disubstituted amino, hydroxy, carboxy, aryl, lower alkoxy, amido, alkoxycarbonyl, tetrahydrofuran-2-yl, hydroxyalkoxy, or sulfonamido, and their isomeric forms.

The term "aryl" as used herein means a monocyclic aromatic ring, or a 9 to 14 membered bicyclic or tricyclic ring system in which at least one ring is aromatic in nature, and includes carbocycles, and heterocycles having one or two heteroatoms chosen from nitrogen, oxygen, and sulfur. Examples of aryl groups include, but are not limited to, phenyl, thiophene, naphthalene, acenaphthene, anthracene, phenanthrene, quinoline, isoquinoline, 1,2,3,4-tetrahydroquinoline, indole, 2,3-dihydroindole, 1H-benzo[b]azepine, 2,3,4,5-tetrahydro-1H-benzo[b]azepine, 2H-benzo[1,4]oxazine, 3,4-dihydro-2H-benzo[1,4]oxazine, 1H,3H-benzo[de]isochromene, 6,7,8,9-tetrahydro-5-oxa-9-benzocycloheptane, 2,3-dihydro-1,4-benzodioxane, and the like.

The term "treatment" as used herein covers any treatment of a disease in a mammal, particularly a human, and includes:

(i) preventing the disease from occurring in a subject which may be predisposed to the disease but has not yet been diagnosed as having it;
(ii) inhibiting the disease, i.e., arresting its development; or
(iii) relieving the disease, i.e., causing regression of the disease.

The term "disease state which can be alleviated by treatment with a serotonin receptor antagonist and/or a histamine receptor antagonist" as used herein is intended to cover all disease states which are generally acknowledged in the art to be usefully treated with compounds having affinity for serotonin receptors and/or such disease states which up to now and currently are treated with histamine receptor antagonists. The preferred disease states, which can be alleviated by serotonin receptor antagonists are those which can be alleviated by $5HT_2$ receptor antagonists. Particularly by the compounds of the present invention diseases can be treated which can be alleviated by a $5\text{-}HT_{2B}$ receptor antagonist and/or up to now and currently are treated by histamine receptor antagonists. As diseases which can be treated by a $5\text{-}HT_{2B}$ receptor antagonist particularly preferred are those disease states which can be usefully treated by a compound with the general formula

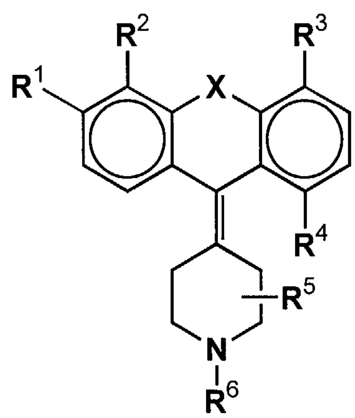

wherein $R^1$, $R^2$, $R^3$, $R^4$, $R^5$ and $R^6$ are defined as above and X is S, N or Se, or a pharmaceutically acceptable salt

thereof, or by one of the pyrimidine compounds mentioned above and/or FMH. Such disease states include, but are not limited to, migraine, pain (e.g. acute, chronic, neuropathic, inflammatory and cancer pain) hypertension, disorders of the gastrointestinal tract (e.g., irritable bowel syndrome, hypertonic lower esophageal sphinter, motility disorders, inflammatory disorders), restenosis, asthma and obstructive airway disease, prostate hyperplasia (e.g., benign prostate hyperplasia), and priapism, allergic disorders, e.g. seasonal and perennial rhinitis, the symptoms of allergic asthma and urticaria like e.g. chronic idiopathic urticaria or physical urticaria.

Particularly the diseases which up to know and currently are treated by histamine receptor antagonists and serotonin receptor antagonists can be treated by the combination of compounds of the present invention, providing a long term effect of alleviation with low sedative effects. Particularly according to the present invention serotonin receptor antagonists currently used for the treatment of diseases are substituted by the selective serotonin receptor antagonists of the present invention and instead of using unselective histamine receptor antagonists the present invention teaches the use of a selective HDC inhibitor.

Although US-A-3,275,640 describes the antihistaminic and/or antiserotonin properties of the class of substituted 1-hydrocarbyl-4-(9H-thioxanthene-9-ylidene)-piperidines in general it neither discloses one of the piperidine derivatives of the present invention nor correlates a selected derivative with a special antihistaminic and/or antiserotonin property. The term "antiserotonin property" is indeed a very broad term and refers to 14 different receptor subtypes. Even more US-A-3,275,640 does not give any hint that a member of the class of substituted 1-hydrocarbyl-4-(9H-thioxanthene-9-ylidene)-piperidines has a selective affinity for one of the various 5-HT receptor subtypes, namely the human 5-$HT_{2B}$ receptor.

The term "antihistaminergic properties" is again a broad term and refers to 4 different receptor subtypes. US-A-3,275,640 does not consider whether a member of the class of substituted 1-hydrocarbyl-4-(9H-thioxanthene-9-ylidene)-piperidines has affinity for one of the various Histamine receptor subtypes.

The property of the novel pyrimidine and piperidine derivatives as a 5-$HT_{2B}$-receptor antagonist provides the possibility for a more specific treatment of the above-cited disease states and reduction of eventually undesired side effects at the same time.

The combination of a histidine decarboxylase inhibitor with one of the serotonin receptor antagonists described in the present application supports the long-term effectiveness of a medicament affecting Histamine receptor subtypes. A medicament containing this combination, however, has a less sedative effect than known traditional medicaments containing only a histamine receptor antagonist.

A preferred histidine decarboxylase inhibitor in the present invention is (S)-$\alpha$-fluoromethylhistidine (FMH)

Repeated administration of FMH revealed a strong reduction in tissue histamine content but only very mild sedative effects (Duggan et al. 1984, Biochem. Pharmacol. 33, 4003-4009; Granus et al. 1985, Agents Actions 16, 244-248; Pikorn et al. 19987, Allergy 42, 496-501; Fukuda et al. 1988, Agents Actions 24,56-64), which is in contrast to most of the histamine antagonists used as anti-allergic agents (Peggs et al. 1995, Am. Pys.Clin. Pharmaco. ).
HDC-deficient mice showed a highly reduced number of peritoneal mast cells (Wiener et al. 2001, Inflamm.Res.50 Suppl.2, 55-56; and mast cells with a reduced number of granules reflecting the lack of histamine as major component of those granules (Ohtsu et al. 2001, FEBS Letters 502, 53-56).

FMH may be prepared in accordance with methods of synthesis well known in the art. For example, the fluorodehydroxylation method in which the $\alpha$-hydroxymethylhistidine is treated with $SF_4$ in liquid HF may be used. Details of this method are further described in US Patent 4,325,961.

One general method for preparing substituted 1-hydrocarbyl-4-(10-thioxanthylidene)-piperidines - not including the novel compounds of the present invention - is described in US-A-3,275,640.

The methods used to synthesis the novel compounds is as follows: A "top" ketone 1 was reacted with a "bottom" Grignard reagent 2 to produce the coupled alcohol 3. The alcohol 3 was dehydrated with formic acid or other acid to produce the desired alkene 4.

Scheme I:

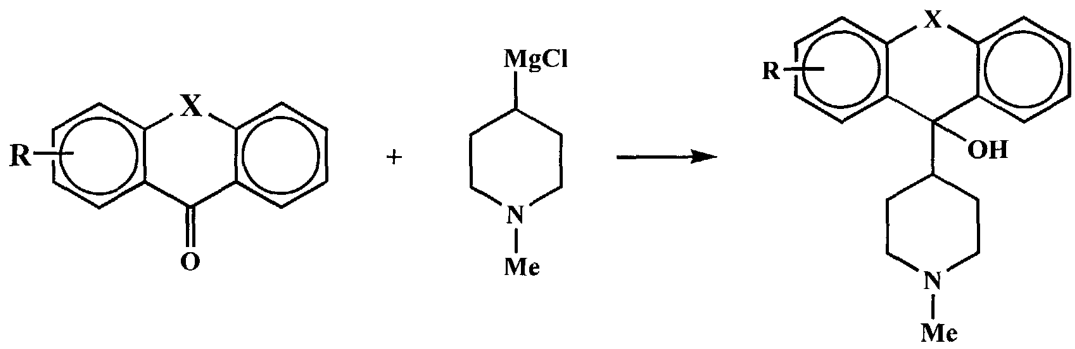

1 2 3

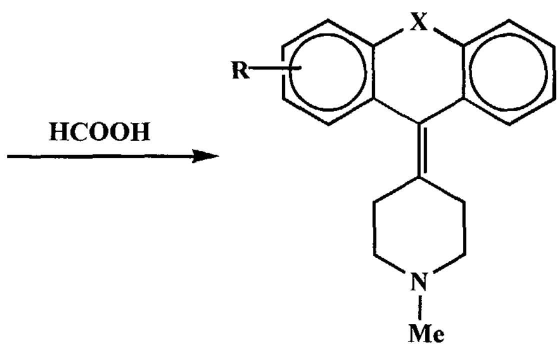


4

One way to synthesize the educt 1, i.e. 3-ethoxythioxanthone, starts from 3-methoxythioxanthone 8a that may be prepared by a procedure described by I. Cervena, J. Metysova, E. Svatek, B. Kakac, J. Holubek, M. Hrubantova, and M. Protiva, in Coll. Czech. Chem. Comm. 41, 881-904 (1978) (Scheme II). 3-Methoxythiophenol 5 is reacted with 2-iodobenzoic acid 6 in a boiling solution of KOH in the presence of copper. After addition of hydrochloric acid the coupled acid 7 is obtained. The acid 7 is cyclized with polyphosphoric acid to produce a mixture of the isomeres 3-methoxythioxanthone 8a and 1-methoxythioxanthone 8b that can be separated by chromatography, preferably column chromatography. Other separation technologies may be used on an industrial scale to manufacture and separate the product, including, but not limited to by example Simulated Moving Bed Separations, Capillary Electrophoresis, High Troughput High Pressure Multi or Single Column Separation methods using chiral or non chiral support and separations media. Enzymatic dehydration in combination with any of the aforementioned technologies or enzyme enrichment separation methods used separately or in combination with any above can also be used.

Scheme II:

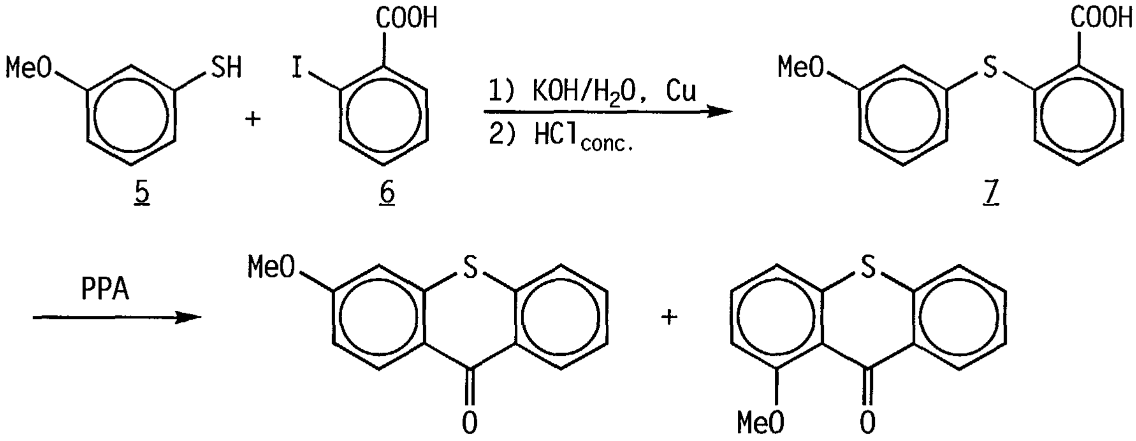


5 6 7

8a 8b

For variation of the side chain of the thioxanthene the separated 3-methoxythioxanthene 8a can be transferred for example to 3-hydroxy thioxanthene 9 by treatment with hydrobromic acid and acetic acid. The 3-hydroxy thioxanthene 9 can then be reacted with iodoethane in the presence of a base, preferably $K_2CO_3$, to produce 3-ethoxythioxanthone 1 according to Scheme III:

Scheme III:

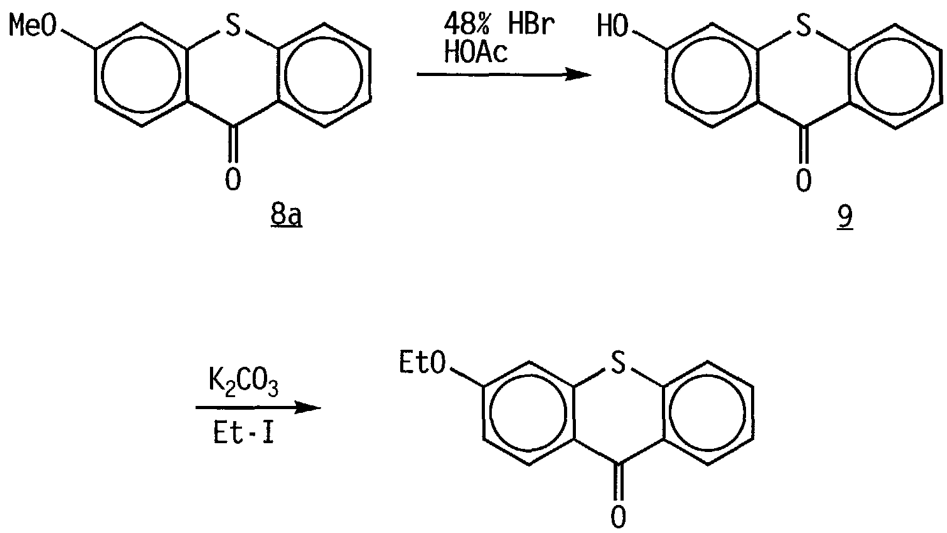


8a 9

1

This compound is coupled with the Grignard reagent 2 prepared from 1-methyl-4-halopiperidine, preferably 1-methyl-4-chloropiperidine to obtain 1-methyl-4-(3-ethoxy-9H-thioxanthene-9-ylidene)-piperidine by Grignard reaction described above (Scheme I).

The other derivatives like 4-(6-Isopropyl-1-methoxy-thioxanthen-9-yliden)-1-methyl-piperidine, 4-(6-Isopropyl-1-hydroxy-thioxanthen-9-yliden)-1-methyl-piperidine, 4-(6-Ethoxy-1-ethyl-thioxanthen-9-yliden)-1-methyl-piperidine, 4-(6-Ethoxy-1-methoxy-thioxanthen-9-yliden)-1-methyl-piperidine, 4-(6-Ethoxy-1-hydroxy-thioxanthen-9-yliden)-1-methyl-piperidine, 4-(6-Dimethylamino-1-methoxy-thioxanthen-9-yliden)-1-methyl-piperidine, 4-(6-Dimethylamino-1-hydroxy-thioxanthen-9-yliden)-1-methyl-piperidine, 4-(3-Ethoxy-selenoxanthen-9-yliden)-1-methyl-piperidine, 4-(3-Ethoxy-1-methoxy-selenoxanthen-9-yliden)-1-methyl-piperidine, 4-(3-Ethoxy-1-hydroxy-selenoxanthen-9-yliden)-1-methyl-piperidine, 3-Ethoxy-9-(1-methyl-piperidine-4-ylidene)-9,10-dihydro-acridine, 6-Ethoxy-1-methoxy-9-(1-methyl-piperidine-4-ylidene)-9,10-dihydro-acridine, 6-Ethoxy-1-hydroxy-9-(1-methyl-piperidine-4-ylidene)-9,10-dihydro-acridine, 4-(3-Ethoxy-5-ethyl-thioxanthen-9-yliden)-1-methyl-piperidine, 4-(3-Ethoxy-4-methyl-thioxanthen-9-yliden)-1-methyl-piperidine, 4-(3-Ethoxy-4-ethyl-thioxanthen-9-yliden)-1-methyl-piperidine and 4-(3-Ethoxy-thioxanthen-9-yliden)-1,3-dimethyl-piperidine, 4-(3,4-(Cyclopent-3'-oxy-1'-eno)-thioxanthen-9-yliden)-1-methyl-piperidine, 4-(3,4-(Cyclopent-4'-oxy-1'-eno)-thioxanthen-9-yliden)-1-methyl-piperidine and 4-(3,4-(Cyclopent-5'-oxy-1'-eno)-thioxanthen-9-yliden)-1-methyl-piperidine may be synthesized accordingly based on the method of Cervená, I. et al as described in Collection Czechoslov. Chem. Cumm. 41 (1978), 881-904 or by one of the methods of Watanabe, M. et al as described in Chem. Pharm. Bull 32 (1984), 1264-1267, Chem. Pharm. Bull 34 (1986), 2810-2820 and Chem. Pharm. Bull 37 (1989), 36-41.

Preparation methods for the pyrimidine compounds of the present invention are described in detail in US-A 5,863,924.

The serotonin receptor antagonist compounds of this invention are selective human 5-$HT_{2B}$ receptor antagonists. Affinity for the 5-$HT_{2B}$ receptors was demonstrated using an in vitro binding assay utilizing cloned human 5-$HT_{2B}$ receptors radiolabelled with [$^3$H]-5HT, as shown in the examples. Selectivity for the human 5-$HT_{2B}$ receptor was shown by counter screening at human 5-$HT_{2A}$ and 5-$HT_{2C}$ receptors. Antagonist properties were determined in rat stomach fundus longitudinal muscle.

Further the compounds of the present invention have selective affinity to only one of the histamine receptor. Affinity for the human $H_1$ receptor was demonstrated using an in vitro binding assay utilizing cloned human $H_1$ receptors radiolabelled with [$^3$H]-mepyramine, as shown in the examples. Selectivity for the human $H_1$ receptor was shown by counter screening at human $H_2$, $H_3$ and $H_4$ receptors. Antagonist properties were determined by [$^3$H]Inositol phosphate production in transiently transfected HEK-293 cells.

Accordingly, the serotonin receptor antagonist compounds of this invention are useful for treating diseases which can be ameliorated by blockade of 5-$HT_{2B}$ receptors. Because of the similarities in the pharmacology of ligand interactions at 5-$HT_{2C}$ and 5-$HT_{2B}$ receptors many of the therapeutic targets that have been proposed for 5-$HT_{2C}$ receptor antagonists are also targets for 5-$HT_{2B}$ receptor antagonists. In particular, several clinical observations suggest a therapeutic role for 5-$HT_{2B}$ receptor antagonists in the prevention of migraine, in that mobilization of 5-HT into the plasma is believed to be a precipitating factor in migraine. Additionally, non-selective 5-$HT_{2B}$ receptor agonists provoke migraine attacks in susceptible individuals, and non-selective 5-$HT_{2B}$ receptor antagonists are effective in preventing the onset of migraine (see Kalkman, Life Sciences 1994, 54, 641-644). It is speculated that activation of 5-$HT_{2B}$ receptors located on endothelial cells of meningeal blood vessels triggers migraine attacks through the formation of nitric oxide (see Schmuck et al., Eur. J. Neurosci. 1996, 8, 959-967). Activation of human $H_1$ receptors induces a nitric oxide-mediated headache in healthy individuals (see Lassen et al., Neuroreport 1995, 31, 1475-1477. Analogously, human cerebral arteries have also been shown to express endothelial $H_1$ and $H_2$ receptors, the former subtype mediating vasodilatation (see Ottosson et al., Br. J. Pharmacol. 1988, 94, 901-907.

Experimental evidence indicates that the compounds of the present invention are useful in the treatment of pain, including acute, chronic, neuropathic, inflammatory, and cancer pain, particularly inflammatory pain. 5-HT (serotonin) plays a key role in the regulation of transmission of nociceptive information at various levels of the peripheral and central nervous systems. (See Richardson, B. P., "Serotonin and Pain", Ann. N.Y. Acad. Sci., 1990, 600, 511-520). Moreover, neuronal systems counting 5-HT are involved not only in the regulation of nociceptive input at the spinal and supraspinal level, but in mediating the nociceptive action of other analgesics including the opiates. 5-HT is a mediator of sensitization of nerve terminal nociceptors that may occur in the genesis of pain associated with inflammation. The 5-$HT_{2B}$ receptor is highly sensitive to activation by 5-HT and specific blockade by selective 5-$HT_{2B}$ receptor antagonists may provide a novel avenue toward analgesia therapy.

Experimental evidence supports a therapeutic role for 5-$HT_{2B}$ receptor antagonists in treating hypertension. In hypertension, one of the most profound increases in vascular responsiveness is observed for serotonin. Two lines of evidence imply that this results from a switch in the receptor mediating vasoconstriction from predominantly 5-$HT_{2A}$ to predominantly 5-$HT_{2B}$. First, serotonin induced contractions of isolated blood vessels from hypertensive animals become resistant to block by selective 5-$HT_{2A}$ receptor antagonists, but remain sensitive to non-selective 5-$HT_{2B}$ receptor antagonists. Second, there is an increase in 5-$HT_{2B}$ receptor mRNA in vessels from hypertensive animals (see Watts et al., J. Pharmacol. Exp. Ther. 1996, 277, 1103-13 and Watts et al., Hypertension 1995, 26, 1056-1059). This hypertension-induced shift in the population of receptor subtype mediating constrictor responses to 5-HT suggests that selective block of vasoconstrictor 5-$HT_{2B}$ receptors may be of therapeutic benefit in the treatment of hypertension.

Clinical and experimental evidence support a therapeutic role for 5-$HT_{2B}$ receptor antagonists in treating disorders of the gastrointestinal tract, in particular irritable bowel syndrome (IBS). Although the pathology underlying IBS remains unclear, there is a well-established implied role for the involvement of serotonin. Thus, meals with a high serotonin content can exacerbate symptoms in some patients (see Lessorf, Scand. J. Gastroenterology 1985, 109, 117-121), while in pre-clinical studies, serotonin has been shown directly to sensitize visceral sensory neurons resulting in an enhanced pain response similar to that observed in IBS (see Christian et al., J. Applied Physiol. 1989, 67, 584-591 and Sanger et al., Neurogastroenterology and Motility 1996, 8, 319-331). The possibility that 5-$HT_{2B}$ receptors play a crucial role in the sensitizing actions of serotonin are suggested by several lines of evidence. Firstly, 5-$HT_{2B}$ receptors

are present in the human intestine (see Borman et al., Brit. J. Pharmacol. 1995, 114, 1525-1527 and Borman et al., Ann. of the New York Acad. of Sciences 1997, 812, 222-223). Secondly, activation of 5-$HT_{2B}$ receptors can result in the production of nitric oxide, an agent capable of sensitizing sensory nerve fibers (see Glusa et al., Naunyn-Schmied. Arch. Pharmacol. 1993, 347, 471-477 and Glusa et al., Brit. J. Pharmacol. 1996, 119, 330-334). Thirdly, poorly selective drugs which display high affinity for the 5-$HT_{2B}$ receptor are clinically effective in reducing the pain associated with IBS and related disorders (see Symon et al., Arch. Disease in Childhood 1995, 72, 48-50 and Tanum et al., Scand. J. Gastroenterol. 1996, 31, 318-325). Together these findings suggest that a selective 5-$HT_{2B}$ receptor antagonist will attenuate both the gastrointestinal pain and abnormal motility associated with IBS.

Clinical and experimental evidence support a therapeutic role for 5-$HT_{2B}$ receptor antagonists in treating restenosis. Angioplasty and bypass-grafting are associated with restenosis which limits the efficacy of these procedures. Platelet-rich thrombus formation is the predominant cause of acute occlusion whereas serotonin, among other platelet-derived mediators, is thought to contribute to late restenosis (see Barradas et al., Clinica Chim. Acta 1994, 230, 157-167). This late restenosis involves proliferation of the vascular smooth muscle. Two lines of evidence implicate a role for 5-$HT_{2B}$ receptors in this process. Firstly, serotonin displays a potent mitogenic activity in cultured smooth muscle and endothelial cells via activation of 5-$HT_2$ receptors (see Pakala et al., Circulation 1994, 90, 1919-1926). Secondly, this mitogenic activity appears to be mediated via activation of a tyrosine kinase second messenger pathway involving mitogen activated protein kinase (MAPK) (see Lee et al., Am. J. Physiol. 1997, 272(1 pt 1), C223-230 and Kelleher et al., Am. J. Physiol. 1995, 268(6 pt 1), L894-901). The recent demonstration that 5-$HT_{2B}$ receptors couple to MAPK (see Nebigil et al., Proc. Natl. Acad Sci. U.S.A. 2000, 97, 22591-2596), coupled with the high affinity of serotonin for this receptor subtype, indicates that a selective 5-$HT_{2B}$ receptor antagonist may afford protection against restenosis of autografted blood vessels or of vessels following angioplasty.

Clinical and experimental evidence support a therapeutic role for 5-$HT_{2B}$ receptor antagonists in treating asthma and obstructive airway disease. Abnormal proliferation of airways smooth muscle, together with hyper-reactivity of the smooth muscle to constrictor stimuli including serotonin, plays a significant role in the pathogenesis of human airway disease such as asthma and bronchial pulmonary dysplasia (see James et al., Am. Review of Respiratory Disease 1989, 139, 242-246 and Margraf et al., Am. Review of Respiratory Disease 1991, 143, 391-400). In addition to other subtypes of serotonin receptor, 5-$HT_{2B}$ receptors are present in bronchial smooth muscle (see Choi et al., Febs Letters 1996, 391, 45-51) and have been shown to stimulate smooth muscle mitogenesis in airways smooth muscle (see Lee et al., Am. J. Physiol. 1994, 266, L46-52). Since elevated concentrations of circulating free serotonin are closely associated with clinical severity and pulmonary function in symptomatic asthmatics, serotonin may play an important role in the pathophysiology of acute attacks (see Lechin et al., Ann. Allergy, Asthma, Immunol. 1996, 77, 245-253). These data suggest that an antagonist of 5-$HT_{2B}$ receptors in airways smooth muscle may therefore be useful in preventing airways constriction resulting from the elevated levels of circulating serotonin and prevent proliferation of the airways smooth muscle that contributes to the long-term pathology of this disease.

Experimental evidence supports a therapeutic role for 5-$HT_{2B}$ receptor antagonists in treating prostate hyperplasia. Obstruction of the urinary tract can occur as a result of prostate hyperplasia and excessive prostate constriction of the urethra. This in turn leads to diminished urinary flow rates and an increased urgency and frequency of urination. 5-$HT_{2B}$ receptors are present in the human prostrate (see Kursar et al., Mol. Pharmacol. 1994, 46, 227-234) and a receptor with the pharmacological attributes of this receptor subtype mediates contraction of the tissue (see Killam et al., Eur. J. Pharmacol. 1995, 273, 7-14). Some drugs effective in the treatment of benign prostate hyperplasia block 5-HT mediated contractions of the prostate (see Noble et al., Brit. J. Pharmacol. 1997, 120, 231-238). 5-$HT_{2B}$ receptors mediate smooth muscle and fibrotic hyperplasia (see Launay et al., J. Biol. Chem. 1996, 271, 3141-3147) and serotonin is mitogenic in the prostate (see Cockett et al., Urology 1993, 43, 512-519), therefore a selective 5-$HT_{2B}$ receptor antagonist may have utility not only in mitigating the excessive prostate constriction, but also in preventing progression of tissue hyperplasia.

Experimental evidence supports a therapeutic role for 5-$HT_{2C}$ receptor antagonists in treating priapism (see Kennett, Curr. Opin. Invest. Drugs 1993, 2, 317-362). MCPP produces penile erections in rats, which effect is blocked by non-selective 5-$HT_{2C/2A}$ receptor antagonists but not by selective 5-$HT_{2A}$ receptor antagonists (see Hoyer, Peripheral actions of 5-HT 1989, Fozard J. (ed.), Oxford University Press, Oxford, 72-99). This therapeutic target for 5-$HT_{2C}$ receptor antagonists is equally a target for 5-$HT_{2B}$ receptor antagonists.

Experimental evidence supports a therapeutic role for histamine $H_1$ receptor antagonists in treating seasonal and perennial allergic rhinitis. The symptomatology of immediate-type allergic diseases, including allergic rhinitis, presumably results from the antigen-induced release of various pharmacologically active substances from mast cells, and from basophilic leukocytes. The substances thus released from these cells, and possibly others as well, are referred to as primary mediators of anaphylaxis and include, among others, histamine. The acute seasonal form of allergic rhinitis, hay fever, and perennial allergic rhinitis are characterized by sneezing, rhinorrhea, nasal congestion, pruritus, conjunctivitis and pharyngitis. In acute seasonal rhinitis, the nose, roof of the mouth, eyes and pharynx often itch, and lacrimation, sneezing and clear, watery nasal discharge follow the pruritus. In perennial rhinitis, chronic nasal obstruction is often prominent and may entend to eustachian tube obstruction. For most patients, topical corticosteroids, some aerosol vasoconstrictor agents, and long acting antihistamine $H_1$ receptor antagonists provide significant relief of symptoms. However, it is commonly known that histamine antagonists have a distinct sedative effect and they involve the drawback of only a short term effect.

Experimental evidence supports a therapeutic role for histamine $H_1$ receptor antagonists in treating allergic pulmonary disease and particular in treating symptoms of allergic bronchial asthma. Patients who suffer from allergic bronchial asthma develop such clinical symptoms as wheezing and dyspnea after exposure to allergens, environmental irritants, viral infections, cold air and exercise. Many of the symptoms result from smooth muscle contraction and vascular dilatation, which, in turn, result from mediator release when the antogen reacts with the IgE antibody on the surface of a mast cell or basophil. This served as a basis for the use of histamine $H_1$ receptor antagonists, which drawbacks are mentioned above.

Experimental evidence supports a therapeutic role for histamine $H_1$ receptor antagonists in treating chronic idiopathic urticaria and some types of physical urticaria. Urticaria is characterized by local wheals and erythema in the dermis; acute urticaria is essentially an anaphylaxis that is limited to the skin and subcutaneous allegy, insect sting, or the like, and is distinct from chronic or idiopathic uricaria which may last several weeks and only rarely be associated with a specific cause. Because these urticarias appear in many cases to be IgE antibody mediated, many of the symptoms may be treated with a $H_1$ receptor antagonist. In treatment of these diseases the short term effect of the histamine receptor antagonists is particularly disadvantageous. Medicaments have to be taken frequently and the sedative effect is increased.

In applying the compounds of this invention to treatment of the above conditions, administration of the active compounds and salts described herein can be via any of the accepted modes of administration, including oral, parenteral and otherwise systemic route of administration. Any pharmaceutically acceptable mode of administration can be used, including solid, semi-solid or liquid dosage forms, such as, for example, tablets, suppositories, pills, capsules, powders, liquids, suspensions, or the like, preferably in unit dosage forms suitable for single administration of precise dosages, or in sustained or controlled release dosage forms for the prolonged administration of the compound at a predetermined rate. The compositions will typically include a conventional pharmaceutical carrier or excipient and a histidine decarboxylase inhibitor and a serotonin receptor antagonist or the pharmaceutically acceptable salts thereof and, in addition, may include other medicinal agents, pharmaceutical agents, carriers, adjuvants, etc.

The amount of the combination of the compounds administered will of course, be dependent on the subject being treated, the severity of the affliction, the manner of administration and the judgment of the prescribing physician. However, an effective dose for oral, parenteral and otherwise systemic routes of administration is in the range of 0.01-20 mg/kg/day, preferably 0.1-10 mg/kg/day for the piperidine and pyrimidine derivatives. For an average 70 kg human, this would amount to 0.7-1400 mg per day, or preferably 7-700 mg/day of the piperidine or pyrimidine derivatives. The histidine decarboxylase inhibitor, particularly $\alpha$-fluoromethylhistidine (FMH)is administered in a range of 0,1 to 25 mg/kg body weight/day, preferably from 0,5 to 15 mg/kg. Expressed in other terms, but reflecting the same dosage levels, the amount administered in a single day will be from 10 mg to 2 g, preferably from 50 mg to 1 g per day.

One of ordinary skill in the art of treating such diseases will be able, without undue experimentation and in reliance upon personal knowledge and the disclosure of this application, to ascertain a therapeutically effective amount of the serotonin receptor antagonists for a given disease.

One of ordinary skill in the art of treating such diseases will be able, without undue experimentation and in reliance upon personal knowledge and the disclosure of this application, to ascertain a therapeutically effective amount of the HDC inhibitor FMH for a given disease.

For solid compositions, conventional non-toxic solid carriers include, for example, pharmaceutical grades of mannitol, lactose, cellulose, cellulose derivatives, sodium crosscarmellose, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. The composition or formulation to be administered will, in any event, contain a quantity of the active compound(s) in an amount effective to alleviate the symptoms of the subject being treated.

Dosage forms or compositions containing the piperidine derivatives and FMH in the range of 0.25 to 95% by weight each with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, cellulose,

cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1 to 95 % by weight of the compounds of the present invention, more preferably 2 to 50 % by weight, most preferably 5 to 8 % by weight.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

Transdermal or "pulsed" transdermal administration may be supported by cremes, gels, dispersions and the like.

A more recently devised approach for parenteral administration employs the implantation of a slow-release or sustained-release system, such that a constant level of dosage is maintained (see, e.g., US-A-3,710,795).

The percentage of active compound contained in such parental compositions is highly dependent on the specific nature thereof, as well as the activity of the compound and the needs of the subject. However, percentages of the piperidine or pyrimidine compounds of the present invention of 0.1 to 10 % by weight in solution are employable, and will be higher if the composition is a solid which will be subsequently diluted to the above percentages. Preferably the composition will comprise 0.2 to 2 % by weight of said compounds in solution. Histidine decarboxylase inhibitor like FMH is contained in such a composition in the same range.

In applying the compounds of the invention to treatment of diseases or disorders of the eye which are associated with an abnormally high intraocular pressure, administration may be achieved by any pharmaceutically acceptable mode of administration which provides adequate local concentrations to provide the desired response. These include direct administration to the eye via drops and controlled release inserts or implants, as well as systemic administration as previously described.

Drops and solutions applied directly to the eye are typically sterilized aqueous solutions containing 0.1 to 10 % by weight, preferably 0.5 to 1 % by weight of the piperidine or pyrimidine compounds and 0.1 to 10 % by weight, preferably 0.5 to 1 % by weight of the histidine decarboxylase inhibitor, along with suitable buffer, stabilizer, and preservative. The total concentration of solutes should be such that, if possible, the resulting solution is isotonic with the lacrimal fluid (though this is not absolutely necessary) and has an equivalent pH in the range of pH 6-8. Typical preservatives are phenyl mercuric acetate, thimerosal, chlorobutanol, and benzalkonium chloride. Typical buffer systems and salts are based on, for example, citrate, borate or phosphate; suitable stabilizers include glycerin and polysorbate 80. The aqueous solutions are formulated simply by dissolving the solutes in a suitable quantity of water, adjusting the pH to about 6.8-8.0, making a final volume adjustment with additional water, and sterilizing the preparation using methods known to those in the art.

The dosage level of the resulting composition will, of course, depend on the concentration of the drops, the condition of the subject and the individual magnitude of responses to treatment. However, a typical ocular composition could be administered at the rate of about 2-10 drops per day per eye of a 0.5 % by weight solution of the compounds of the present invention.

The compositions of the present invention may also be formulated for administration in any convenient way by analogy with other topical compositions adapted for use in mammals. These compositions may be presented for use in any conventional manner with the aid of any of a wide variety of pharmaceutical carriers or vehicles. For such topical administration, a pharmaceutically acceptable non-toxic formulation can take the form of semisolid, liquid, or solid, such as, for example, gels, creams, lotions, solutions, suspensions, ointments, powders, or the like. As an example, the active component may be formulated into a gel using ethanol, propylene glycol, propylene carbonate, polyethylene glycols, diisopropyl adipate, glycerol, water, etc., with appropriate gelling agents, such as Carbomers, Klucels, etc. If desired, the formulation may also contain minor amounts of non-toxic auxiliary substances such as preservatives, antioxidants, pH buffering agents, surface active agents, and the like. Actual methods of preparing such dosage forms are known, or will be apparent, to those skilled in the art; for example, see Remington's Pharmaceutical Sciences, Mack Publishing Company, Easton, Pa., 19th Edition, 1995.

Preferably the pharmaceutical composition is administered in a single unit dosage form for continuous treatment or in a single unit dosage form ad libitum when relief of symptoms is specifically required.

Examples:

Example 1: Preparation of 1-Methyl-4-(3-ethoxy-9H-thioxanthene-9-ylidene)-piperidine and corresponding Hydrochloride Salt

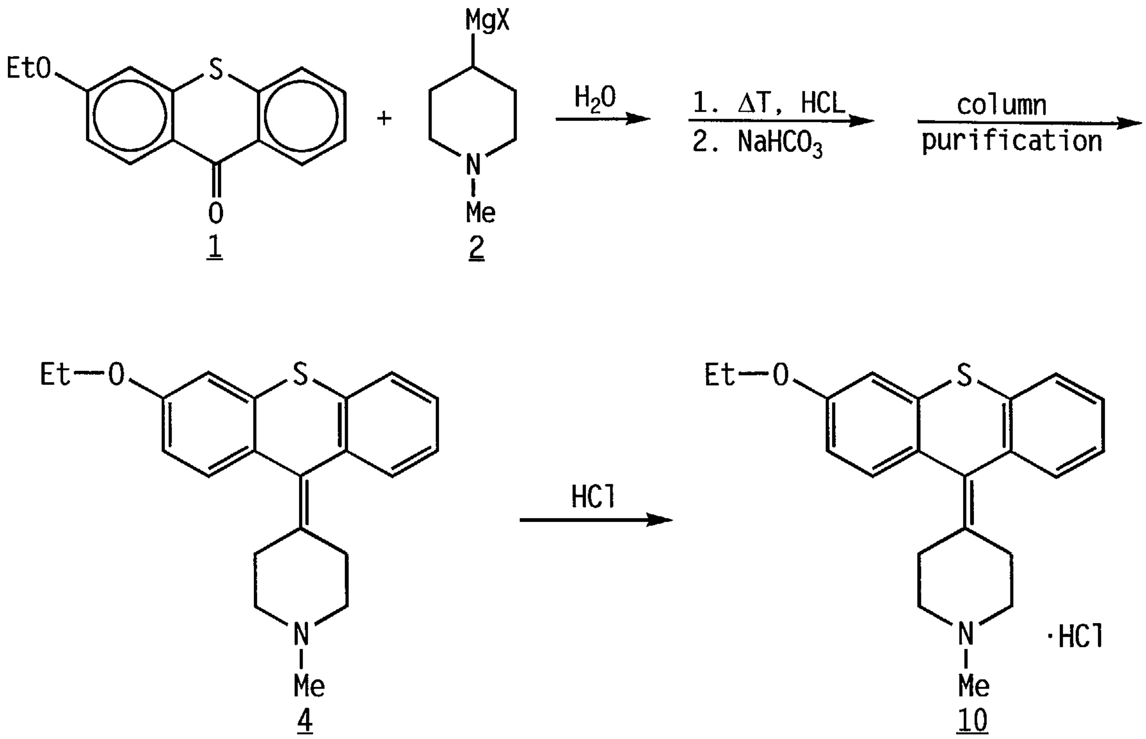


1 2 4 10

0.63 g of the 3-ethoxythioxanthene 1 were dissolved in tetrahydrofuran (THF). The Grignard reagent 2 was added to the solution in one portion. The resulting reaction mixture was stirred under argon at room temperature for 1.5 h until completeness of the reaction. The mixture was quenched with water and extracted three times with ethyl acetate. The combined organic phase was dried over sodium sulfate, filtered and evaporated to dryness to give about 0.75 g of a brown oil, which was then stirred at reflux for about 50 min in concentrated hydrochloric acid until completeness of the reaction. The mixture was allowed to cool to room temperature and then diluted with water and ethyl acetate. While stirring, small increments of sodium hydrogen carbonate were added until no more gas evolved. The organic layer was collected. The aqueous layer was back extracted five times with ethyl acetate. The combined organic phase was dried over sodium sulfate, filtered and evaporated under vacuum on a rotary evaporator at about 40°C to give about 0.67 g of a brown oil, which was then dissolved in 4 to 6 ml of ethyl acetate and absorbed in 2 to 3 g of silica gel. The silica gel was dried on a rotary evaporator until it was freely flowing as a powder. The dried silica gel was loaded onto a column (about 40 g of silica gel) using 5%-10% methanol/dicloromethane. Desired fractions were combined and evaporated to dryness to give 0.52 g of 1-methyl-4-(3-ethoxy-9H-thioxanthene-9-ylidene)-piperidine 4 as a brown oil (yield = 62%).

In order to prepare the hydrochloride salt 10, the brown oil 4 was dissolved in 3 to 5 ml of ethyl acetate, followed by addition of 4 N hydrochloric acid/dioxane into the stirring solution (3 equivalents of 4 N hydrochloric acid, 0.89 ml). The solution turned brown-red as the hydrochloric acid was added. The mixture was further stirred for 10 min and let settled at room temperature for 20 minutes. 2 to 4 ml of heptane were added to the stirring solution and some red-brown oil separated. The solvents (heptane und ethyl acetate) were evaporated off. The red-brown oil was chased two times with heptane to get rid off all other solvents. At the second heptane chase some of the oil became solid. It was continued to evaporate the product to dryness to give a red-brown solid (still partially sticky). Heptane was added to the red-brown solid and the product was triturated for 1.5 h at room temperature. Then, the solid was filtered, washed with heptane and dried under vacuum to give 0.51 g of the hydrochloride salt 10 as a red-brown solid (yield = 87%).

Example 2: Synthesis of 4-(6-Ethoxy-1-methoxy-thioxanthene-9-ylidene)-1-methyl-piperidine

6-Ethoxy-1-methoxythioxanthone 1 is reacted with the Grignard reagent 2 prepared from 1-methyl-4-halopiperidine, preferably 1-methyl-4-chloropiperidine, according to Scheme I. The alcohol 3 is isolated and dehydrated with an acid, preferably hydrochloric acid or formic acid to produce 4-(6-Ethoxy-1-methoxy-thioxanthene-9-ylidene)-1-methyl-piperidine (4).

Scheme I:

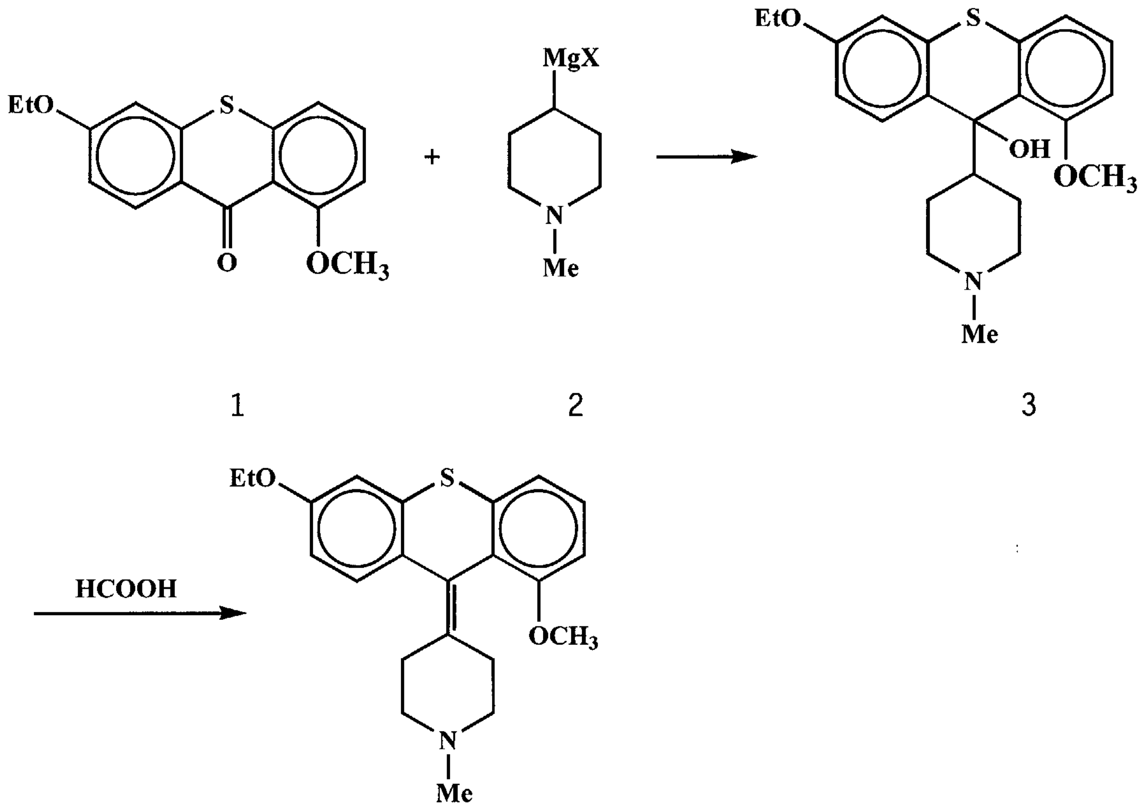


1 2 3

4

One way to synthesize the 6-ethoxy-1-methoxythioxanthone 1 is described by I. Cervena, J. Metysova, E. Svatek, B. Kakac, J. Holubek, M. Hrubantova, and M. Protiva, in Coll. Czech. Chem. Comm. 41, 881-904 (1978) (Scheme II). 3-Ethoxythiol 5 is reacted with 2-iodo-4-methoxybenzoic acid 6 in a boiling aqueous solution of KOH in the presence of copper. After addition of hydrochloric acid the coupled acid 7 is obtained. The acid 7 is cyclized with polyphosphoric acid to produce a mixture of the isomeres 1-methoxy-6-ethoxythioxanthone 8a and 1-methoxy-8-ethoxythioxanthone 8b that can be separated by chromatography, preferably column chromatography.

Scheme II:

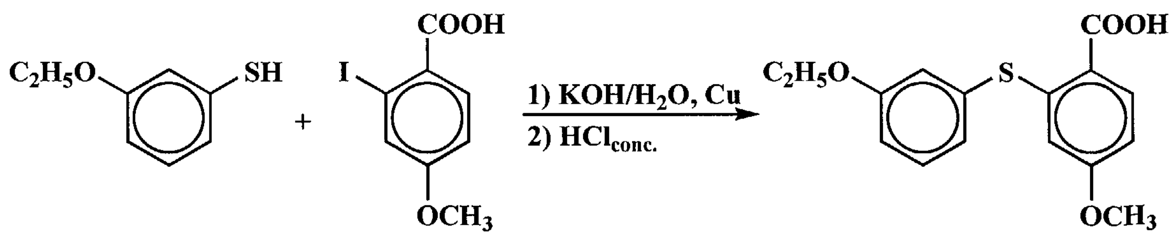


5 6 7

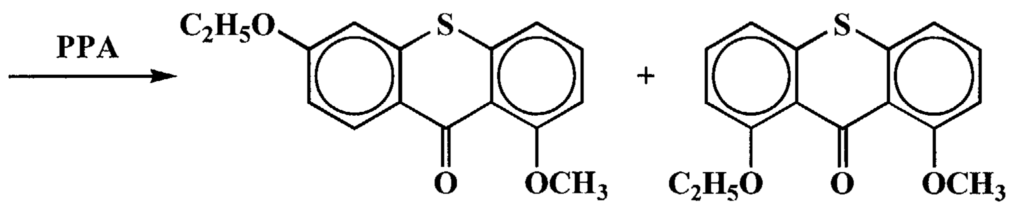


8a 8b

A second procedure to produce the "top" ketone is based on the method of M. Watanabe et al., Chem. Pharm Bull. 37, 36-41 (1989). Starting from 4-isopropylbenzoicacid 10, this is converted to the amide 11 which is treated with strong base to extract the proton adjacent to the amide group and, after the introduction of molecular sulfur and treatment with acid, the thiol group is introduced as in 12. Treatment of the bromoanisole 13 with strong base converts it to a benzyne that reacts with 12 to produce the desired ketone 14.

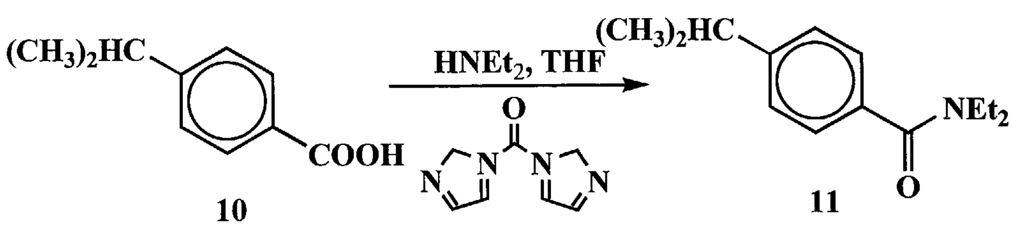


10 11

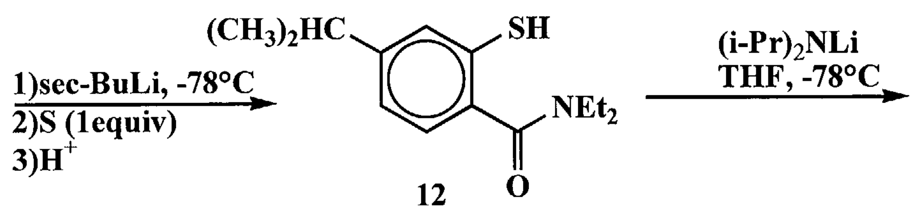


12

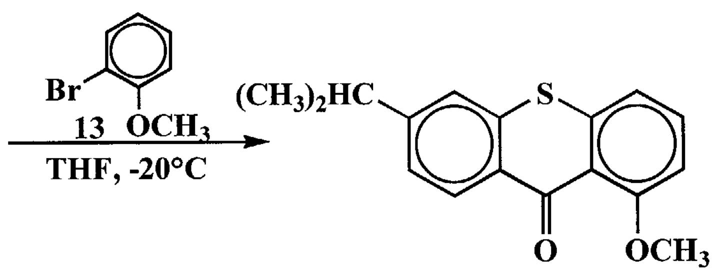


14

Which procedure is suitable for the different compounds of the present invention depends particularly on the availability of the starting materials.

Example 3: Cloned Human 5-$HT_{2B}$ Receptor Binding Assay

The following describes an in vitro binding assay utilizing cloned 5-$HT_{2B}$ receptors radiolabelled with [$^3$H]-5HT.

Receptor Binding Assay

HEK 293 cells transiently transfected with an expression plasmid pXMD1-hu2B encoding the human 5-HT2B receptor (see Schmuck et al., FEBS Lett., 1994, 342, 85-90) were used as described previously (Schmuck et al., Eur. J. Pharmacol., 1996, 8, 959-967). Two days after transfection cells were harvested, pelleted at 500g for 5 min at 4°C, gently resuspended in ice-cold buffer1 (50 mM TRIS pH 7.7, 4 mM $CaCl_2$) and homogenized using a Polytron PT 1200 tissue homogenizer (position 6 for 30 s). Cells were pelleted at 50,000g, 4°C for 10 min, washed with buffer1 and pelleted again. The final pellet was resuspended in incubation buffer (50 mM TRIS pH 7.7, 4 mM $CaCl_2$, 10 μM pargyline and 0.1 % by weight ascorbic acid). The binding assay consisted of 300 μl of membrane suspension (protein concentration = 0.3 to 0.5 mg/ml), 150 μl of competing drug and 50 μl of [$^3$H]5-HT at a final concentration of 4 to 5 nM. The mixture was incubated at 37°C for 30 min and the assay terminated by rapid filtration and two washing steps with 5 ml of cold 20 mM Tris-HCl pH = 7.5, and 154 mM NaCl over Whatman GFB filters. Filters were counted by liquid scintillation. Non-specific binding was determined in the presence of an excess of 5-HT (100 μM). Bound radioligand represented less than 1 % of free radioligand. In competition experiments, specific binding represented about 60 % of total binding. Results expressed as $pK_i$ values are shown in Table 1.

Proceeding as in the example above the piperidine or pyrimidin compounds of the present invention were found to have affinity for the 5-$HT_{2B}$ receptor.

Example 4: 5-$HT_{2A}$ 5-$HT_{2B}$ 5-$HT_{2C}$ Receptor Binding Methods

The following describes receptor binding methods in which ligands with high affinity for 5-$HT_{2B}$ receptors were counter screened at 5-$HT_{2A}$ and 5-$HT_{2C}$ receptors to demonstrate selectivity.

5-$HT_{2A}$ receptors were labeled with [$^3$H]ketanserin in human cortex, in HEK293 cells expressing a cloned human 5-$HT_{2A}$ receptor and in HEK293 cells expressing the rat 5-$HT_{2A}$ receptor. For competition binding studies the ligand concentration was approximately 0.1 nM. For saturation binding studies concentrations of radioligand ranged from 0.01 nM to 2.0 nM. Assays were conducted in 0.5 ml of assay buffer (50 mM Tris-HCl, 4 mM calcium chloride, 0.1 % by weight ascorbic acid) (pH 7.4 at 4°C). Non-specific binding was defined with 10 mM unlabelled ketanserin. After a 60 min incubation at 32°C, membranes were harvested onto filters treated with 0.1 % by weight of polyethylenimine and the bound radioactivity was determined.

Human 5-$HT_{2B}$ receptors were labelled in HEK293 cells as described above. except that the radioligand was [$^3$H]-5HT and that the assay buffer contained pargyline in a concentration of 10 mM and 0.1 % by weight of ascorbic acid. For competition binding studies the radioligand concentration was approximately 0.4 nM while for saturation binding studies the concentration of [$^3$H]-5HT ranged from 0.05 to 8 nM. Non-specific binding was defined with 10 mM 5-HT. Incubations were for 120 min at 4°C.

5-$HT_{2C}$ receptors were labeled in choroid plexus, Cos-7 cells expressing the human 5-$HT_{2C}$ receptor and in NIH-3T3 expressing the rat 5-$HT_{2A}$ receptor.

Assays were conducted as described for the 5-$HT_{2A}$ receptor except that the radioligand was [$^3$H]mesulergine. The radioligand concentration for competition studies was approximately 0.2 nM while for saturation binding studies the concentration ranged from 0.1 to 18 nM. Non-specific binding was defined with 10 μM unlabelled mesulergine.

Competition radioligand binding data was analyzed using a four parameter logistic equation and iterative curve-fitting techniques to obtain estimates of the $IC_{50}$ and Hill slope. Kd values, determined from saturation binding studies were then used to calculate inhibition dissociation constants (Ki).

Proceeding as in the example above the piperidine or pyrimidine compounds of the present invention were found to have affinity for the 5-$HT_{2B}$ receptor. Results are shown in Table 1

Example 5: 5-$HT_{2B}$ Receptor Tissue Based Functional Assay

The following describes an in vitro functional assay characterizing 5-HT receptors (the putative 5-$HT_{2B}$) in rat stomach fundus longitudinal muscle (Baxter et al., Brit. J. Pharmacol. 1994, 112, 323-331).

Strips of longitudinal muscle were obtained from the stomach fundus of male Sprague Dawley rats. The mucosa was removed and the strips were suspended with a resting tension of 1 g in oxygenated (95/% $O_2$ /5% $CO_2$)

Tyrode solution at 37°C. The composition of the Tyrode solution was as follows (mM): NaCl 136.9; KCl 2.7; $NaH_2PO_4$ 0.4; $MgCl_2$ 1.0; glucose 5.6; $NaHCO_3$ 11.9; $CaCl_2$ 1.8.

Concentration-response curves to 5-HT receptor agonists were constructed under conditions where cyclooxygenase activities were inactivated by 3 µm indomethacin, monoamine oxidase activities inactivated by 0.1 mM pargyline, and uptake mechanisms inactivated by 30 µM cocaine and 30 µM corticosterone.

Effects of drugs were monitored by tension transducers and recorded on polygraph recorders. Tissue response was measured as changes in isometric tension (g). The mean potency ($EC_{50}$) and maximum response were evaluated by standard iterative curve fitting procedures.

Effects of antagonists were determined by measuring dextral shifts to the agonist concentration-response curve after equilibration of the antagonists for at least 1 h. Concentration ratios were measured at half maximal response levels and single concentration antagonist affinities were determined by the equation:

$$KB = \frac{\text{antagonist concentrat ion}}{\text{concentrat ion ratio}}$$

Schild regression analysis was employed with multiple antagonist concentrations when the compound showed competitive behavior.

Proceeding as in the example above the piperidine or pyrimidine compounds were found to be antagonists at the 5-$HT_{2B}$ receptor.

Example 6: Cloned Human $H_1$, $H_2$, $H_3$ and $H_4$-Receptor Binding Assay

COS-7 cells were transiently transfected with an expression plasmid pCineohH1, pCineohH2, pCineohH3 and pCineohH4 encoding the human Histamine $H_1$, $H_2$ $H_3$ or $H_4$ receptor, respectively. Transfected cells were harvested after 48 h, homogenized in ice-cold 50 mM $Na_2$/potassium phosphate buffer (pH 7.4) and used for radioligand binding studies. Cell homogenates (40 - 50 µg of protein) were incubated for 30 min at 25°C in 50 mM $Na_2$/potassium phosphate buffer (pH 7.4) in 400 µl with the various concentrations of either [$^3$H]-mepyramine, [$^3$H]-thiotidine, [$^3$H]-R-α-Methylhistamine, and [$^3$H]-pyramilamine for cells expressing recombinant human $H_1$, $H_2$, $H_3$ and $H_4$ receptors, respectively. The nonspecific binding was defined in the presence of 1 µM mianserin. In displacement studies, cell homogenated were incubated either with 1 nM [$^3$H]-mepyramine, 15 nM [$^3$H]-thiotidine, 0.5 nM [$^3$H]-R-α-Methylhistamine, or 15 nM [$^3$H]-pyramilamine and increasing concentrations of competing ligands. The incubations were stopped by rapid dilution with 3 ml of ice-cold 50 mM $Na_2$/potassium phosphate buffer (pH 7.4). The bound radioactivity was separated by filtration through Whatman GF/C filters that had been treated with 0.3 % polyethyleneimine. Filters were washed twice in 3 ml of buffer and radioactivity retained on the filters was measured by liquid scintillation counting.

Proceeding as in the example above the piperidine and pyrimidine compounds as well as FMH were found to have low affinity for the human histamine receptors. Results are shown in Table 1.

Example 7: Human Histamine Receptor Functional Assay

For the measurement of the [$^3$H]-inositol phosphate formation transiently transfected HEK-293 cells were seeded in 24 well plates and labeled to equilibrium with *myo*-[2-$^3$H]-inositol (3 ìCi/ml) for an additional 24 hours in growth medium. The medium was aspirated and cells were washed once with 500 µl HBS-buffer (130 mM NaCl, 900 µM $NaH_2PO_4$, 800 µM $MgSO_4$, 5.4 mM KCl, 1.8 mM $CaCl_2$, 25 mM Glucose in 20 mM HEPES pH 7.4). Two min after applying 20 mM $Li^+$ the cells were stimulated by addition of histamine in HBS-buffer. The incubation was stopped by aspiration off the culture medium and the addition of cold 10 mM formic acid. [$^3$H]inositol phosphates were isolated by anion exchange chromatography (see Seuwen et al., 1988, EMBO J., 7, 161-168). The $pK_B$ value was calculated according to the formula:

$$pK_B = \log (A'/A - 1) - \log [B],$$

where A'/A is the ratio of the agonist concentrations ($EC_{50}$ in the presence / $EC_{50}$ in the absence of antagonist) and [B] the concentration of antagonist.

Proceeding as in the example above the compounds of the present invention were found to be antagonists of lower potency at the human Histamine receptors. Results are shown in Table 1.

Table 1:

| Receptor Affinity: pK Values of the compounds | | | | | | | |
|---|---|---|---|---|---|---|---|
| | measured receptor | | | | | | |
| Compound | 5-$HT_{2A}$ | $5HT_{2B}$ | $5HT_{2C}$ | $H_1$ | $H_2$ | $H_3$ | $H_4$ |
| 4-(6-Ethoxy-1-methoxy-thioxanthen-9-yliden)-1-methyl-piperidine | 8,99 | 10,35 | 7,77 | 8,43 | 5,69 | n.A. | n.A. |
| 4-(6-Ethoxy-1-hydroxy-thioxanthen-9-yliden)-1-methyl-piperidine | 8,82 | 10,94 | 7,86 | 7,44 | 5,49 | n.A. | n.A. |
| 4-(6-Isopropyl-1-methoxy-thioxanthen-9-yliden)-1-methyl-piperidine | 9,13 | 9,77 | 7,79 | 7,98 | 5,98 | n.A. | n.A. |
| 4-(6-Isopropyl-1-hydroxy-thioxanthen-9-yliden)-1-methyl-piperidine | 9,11 | 9,92 | 7,52 | 7,45 | n.d. | n.A. | n.A. |
| 3-Ethoxy-9-(1-methyl-piperidine-4-ylidene)-9,10-dihydro-acridine | 8,93 | 9,13 | 7,43 | 7,53 | n.d. | n.A. | n.A. |
| 4-(6-Methoxy-1-methoxy-thioxanthen-9-yliden)-1-methyl-piperidine | 10,37 | 11,06 | 8,81 | 9,89 | n.d. | n.A. | n.A. |
| FMH | n.d. | n.A. | n.d. | n.A. | n.A. | n.A. | n.A. |
| n.d. = not determined<br>n.A. = no affinity (= no inhibition of histamine activity) detected | | | | | | | |

Example 8: Preparation of α-fluoromethylhistidine

(S)-α-fluoromethylhistidine and esters thereof may be represented by the following structural formula:

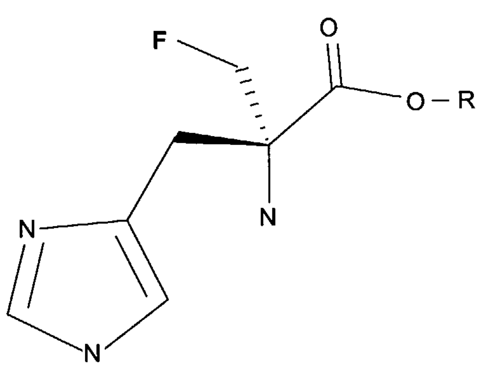

where R is H or a pharmaceutically acceptable ester forming group. (S)-α-fluoromethylhistidine may be prepared in accordance with methods of synthesis well known in the art. For example, the fluorodehydroxylation method in which the the (S)-α-fluoromethylhistidine is treated with SF4 in liquid HF may be used. Details of this method is further described in U:S: Pat.No. 4,325,961.

Example 9: In vivo measurement of Histamine level

The histamine content of organs from treated versus untreated animals were measured by the method described by Ohtsu et al. (1996, J. Biol. Chem. 271,28439-28444). For the histamine assay, organs were homogenized in 10 vol. of 3% perchloric acid, containing 5mM sodium EDTA by a Polytron homogenizer and centrifuged at 10.000xg for 30 min at 4°C. An aliquot of 50 µl of the supernatant was injected into a HPLC system in which histamine was separated on a cation-exchanger column and automatically mixed with o-phthalaldehyde in alkaline conditions. Fluorescence was measured with a fluoromonitor using excitation and emission wavelengths of 360 and 450 nm, respectively.

HDC activity was expressed as the amount of histamine synthesized from histidine per min per mg of protein. Protein was measured with the protein assay system from Bio-Rad.

## Claims

1. Pharmaceutical composition comprising a serotonin receptor antagonist and a histidine decarboxylase inhibitor or their pharmaceutically acceptable salts or esters.

2. Use of a histidine decarboxylase inhibitor and esters and pharmaceutically acceptable salts thereof in combination with a serotonin receptor antagonist or a pharmaceutically acceptable salt thereof for the manufacture of a medicament for the treatment of a disease state which can be alleviated by treatment with a serotonin receptor antagonist and/or histamine receptor antagonist.

3. The composition of claim 1 or the use according to claim 2, whereby the serotonin receptor antagonist is a 5-$HT_2$ receptor antagonist.

4. The composition or the use according to claim 3, whereby the serotonin receptor antagonist is a 5-$HT_{2B}$ receptor antagonist.

5. The composition or use according to claim 3 or 4, whereby the serotonin receptor antagonist has the general formula

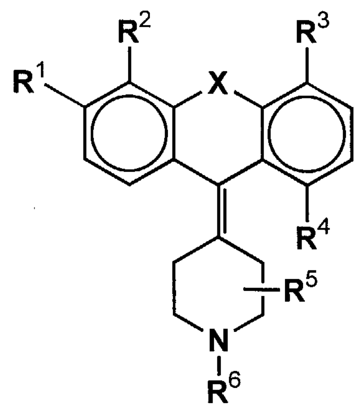

wherein R1 is methyl, ethyl, propyl, isopropyl, butyl, isobutyl, pentyl (straight or branched), hexyl (straight or branched), methoxy, ethoxy, propoxy, isopropoxy, butoxy, isobutoxy, phenoxy, trifluoromethyl, trifluoromethoxy, amino, dimethylamino, fluorine, chlorine, bromine, $-CON(CH_3)_2$ or $-CON(C_2H_5)_2$, R2 is methyl, ethyl, propyl, isopropyl, butyl, isobutyl, pentyl, hexyl, hydroxy or hydrogen, or R1 and R2 are forming a heterocycle, R3 is methyl, ethyl, propyl, isopropyl, butyl isobutyl, pentyl, hexyl, hydroxy or hydrogen, R4 is hydroxy, methoxy, ethoxy, propoxy, isopropoxy, butoxy, isobutoxy, trifluormethyl, amino, dimethylamino, diethylamino, fluorine, chlorine or bromine, methyl, ethyl, propyl, isopropyl, butyl or hydrogen, R5 is methyl or hydrogen, R6 is methyl or ethyl and X is S, N or Se, or a pharmaceutically acceptable salt thereof.

6. The composition or use according to claim 5, whereby R1 is an isopropyl, dimethylamino, methoxy or ethoxy group, R2 is methyl, ethyl or hydrogen, or R1 and R2 are forming a heterocycle, R3 is ethyl or hydrogen, R4 is methoxy, hydroxy, ethyl, methyl or hydrogen, R5 is methyl or hydrogen, R6 is methyl and X is S, N or Se, or a pharmaceutically acceptable salt thereof.

7. The composition or use according to claim 6, whereby the serotonin receptor antagonist is selected from the group of 1-methyl-4-(3-ethoxy-9H-thioxanthene-9-ylidene)-piperidine, 4-(6-Isopropyl-1-methoxy-thioxanthen-9-yliden)-1-methyl-piperidine, 4-(6-Isopropyl-1-hydroxy-thioxanthen-9-yliden)-1-methyl-piperidine, 4-(6-Ethoxy-1-ethyl-thioxanthen-9-yliden)-1-methyl-piperidine, 4-(6-Ethoxy-1-methoxy-thioxanthen-9-yliden)-1-methyl-piperidine, 4-(6-Ethoxy-1-hydroxy-thioxanthen-9-yliden)-1-methyl-piperidine, 4-(6-Methoxy-1-methoxy-thioxanthen-9-yliden)-1-methyl-piperidine, 4-(6-Dimethylamino-1-methoxy-thioxanthen-9-yliden)-1-methyl-piperidine, 4-(6-Dimethylamino-1-hydroxy-thioxanthen-9-yliden)-1-methyl-piperidine, 4-(3-Ethoxy-selenoxanthen-9-yliden)-1-methyl-piperidine, 4-(3-Ethoxy-1-methoxy-selenoxanthen-9-yliden)-1-methyl-piperidine, 4-(3-Ethoxy-1-hydroxy-selenoxanthen-9-yliden)-1-methyl-piperidine, 3-Ethoxy-9-(1-methyl-piperidine-4-ylidene)-9,10-dihydro-acridine, 6-Ethoxy-1-methoxy-9-(1-methyl-piperidine-4-ylidene)-9,10-dihydro-acridine, 6-Ethoxy-1-hydroxy-9-(1-methyl-piperidine-4-ylidene)-9,10-dihydro-acridine, 4-(3-Ethoxy-5-ethyl-thioxanthen-9-yliden)-1-methyl-piperidine, 4-(3-Ethoxy-4-methyl-thioxanthen-9-yliden)-1-methyl-piperidine, 4-(3-Ethoxy-4-ethyl-thioxanthen-9-yliden)-1-methyl-piperidine, 4-(3-Ethoxy-thioxanthen-9-yliden)-1,3-dimethyl-piperidine, 4-(3,4-(Cyclopent-3'-oxy-1'-eno)-thioxanthen-9-yliden)-1-methyl-piperidine, 4-(3,4-(Cyclopent-4'-oxy-1'-eno)-thioxanthen-9-yliden)-1-methyl-piperidine and 4-(3,4-(Cyclopent-5'-oxy-1'-eno)-thioxanthen-9-yliden)-1-methyl-piperidine.

**8.** The composition or use according to claim 3 or 4, whereby the serotonin receptor antagonist has the general formula

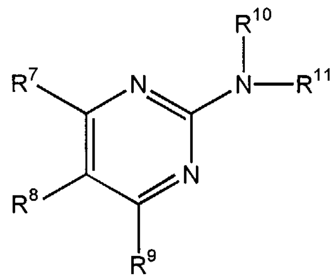

wherein $R^7$ is hydrogen, alkyl, hydroxyalkyl, cycloalkyl, cycloalkyl methyl, ethyl, propyl, isopropyl, tert-butyl, butyl, pentyl, n-hexyl, alkenyl, thioalkoxy with 1 to 6 carbon atoms, halo, fluoroalkyl, --$NR^{12}$ $R^{13}$, --$CO_2R^{14}$, --$O(CH_2)_n$ $R^{15}$, or alkyl substituted with a substituent selected from the group consisting of hydroxy, alkoxy, halo, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl isoquinoline, isoquinolin-1-yl, isoquinolin-4-yl, isoquinolin-8-yl, 3,4-dihydro-2H-quinolin-1-yl-1, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydro-quinoline, 1,2,3,4-tetrahydro-quinolin-1-yl, 1,2,3,4-tetrahydroquinolin-5-yl, indole, indol-1-yl, 1H-indol-4-yl, 1H-indol-7-yl, alkylylindol-3-yl, alkyl-1-H-indol-4-yl, 2,3-dihydroindole, 2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H,3H-benzo[de]isochromene, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, and benzo-1,4-dioxane;
in which
n is 1, 2, or 3;
$R^{12}$ and $R^{13}$, are hydrogen or methyl, ethyl, propyl, isopropyl, tert-butyl, butyl, pentyl or n-hexyl;
$R^{14}$ is hydrogen or methyl, ethyl, propyl, isopropyl, tert-butyl, butyl, pentyl or n-hexyl; and
$R^{15}$ is hydrogen, methyl, ethyl, propyl, isopropyl, tert-butyl, butyl, pentyl or n-hexyl, hydroxy, hydroxy methyl, hydroxy ethyl, propyl, hydroxy isopropyl, hydroxy tert-butyl, hydroxy butyl, hydroxy pentyl or hydroxy n-hexyl, according alkenyl with 1 to 6 carbon atoms, or according alkoxy group with 1 to 6 carbon atoms;
$R^8$ is hydrogen, methyl, ethyl, propyl, isopropyl, tert-butyl, butyl, pentyl or n-hexyl, according alkoxy, halo, or according fluoroalkyl;
$R^9$ is phenyl, alkylyphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl alkoxynaphth-2-yl, halonaphth-2-yl, acenaphthene, acenaphthen-5-yl, acenaphthen-6-yl, alkylacenaphthen-5-yl, anthracene, phenanthrene, phenanthrene-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydroquinoline, 1,2,3,4-tetrahydroquinolin-1-yl, 1,2,3,4-tetrahydroquinolin-5-yl, indole, indol-1-yl, 1H-indol-4-yl, 1H-indol-7-yl, alkylindol-3-yl, alkyl-1H-indol-4-yl, 2,3-dihydroindole, 2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H, 3H-benzo[de]isochromone, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, or benzo-1,4-dioxane;

$R^{10}$ is hydrogen, methyl, ethyl, propyl, isopropyl, tert-butyl, butyl, pentyl or n-hexyl, cycloalkyl, alkenyl, acyl, amino, amido, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydroquinoline, 1,2,3,4-tetrahydroquinolin-1-yl, 1,2,3,4-tetrahydro-quinolin-5-yl, indole, indol-1-yl, 1H-indol-4-yl, 1H-indol-7-yl, alkylindol-3-yl, alkyl-1H-indol-4-yl, 2,3-dihydroindole-2,3-dihydroindol-1-yl, alkyl-2,3-dihydroindol-1-yl, 1H-benzo[b]azepine, 2,3,4,5-tetrahydro-1H-benzo[b]azepine, 2H-benzo[1,4] oxazine, 3,4-dihydro-2H-benzo[1,4]oxazine, 2,3-dihydro-2H-benzo[1,4]oxazin-4-yl, 3,4-dihydro-2H-benzo][1,4] oxazin-1-yl, 3,4-dihydro-2H-benzo[1,4]oxazin-5-yl , 1H,3H-benzo[de]isochromene, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, benzo-1,4-dioxane, $--(CH_2)_mNR^{16}R^{17}$, or methyl, ethyl, propyl , isopropyl, tert-butyl, butyl, pentyl or n-hexyl substituted with a substituent selected from the group consisting of amino, monosubstituted amino, disubstituted amino, hydroxy, carboxy, phenyl, alkylphenyl, alkoxyphenyl, nitrophenyl, halophenyl, 4-amino-5-chloro-2-methoxyphenyl, thiophene, thiophen-2-yl, halothiophen-2-yl, naphthalene, naphth-1-yl, alkylnaphth-1-yl, alkoxynaphth-1-yl, halonaphth-1-yl, naphth-2-yl, alkylnaphth-2-yl, alkoxynaphth-2-yl, halonaphth-2-yl, acenaphthene, acenaphthen-5-yl, acenaphthen-6-yl, alkylacenaphthen-5-yl, anthracene, phenanthrene, phenanthren-9-yl, quinoline, quinolin-2-yl, quinolin-4-yl, quinolin-8-yl, isoquinoline, isoquinolin-1-yl, isoquinolin-4-yl, isoquinolin-8-yl, 3,4-dihydro-2H-quinolin-1-yl, halo-3,4-dihydro-2H-quinolin-1-yl, alkyl-3,4-dihydro-2H-quinolin-1-yl, alkoxy-3,4-dihydro-2H-quinolin-1-yl, 3,4-dihydro-1H-quinolin-2-yl, halo-3,4-dihydro-1H-quinolin-2-yl, alkyl-3,4-dihydro-1H-quinolin-2-yl, alkoxy-3,4-dihydro-1H-quinolin-2-yl, 1,2,3,4-tetrahydro-quinoline, 1,2,3,4,-tetrahydroquinolin-1-yl, 1,2,3,4-tetrahydroquinolin-5-yl, indole, indol-1-yl, 1H-indol-4-yl, 1H-indol-7-yl, alkylindol-3-yl, alkyl-1H-indol-4-yl, 2,3-dihydroindole, 2,3-dihydroindol-1-yl, alkyl-2,3-dihydroindol-1-yl, 1-benzo[b]azepine, 2,3,4,5-tetrahydro-1H-benzo[b] azeplne, 2H-benzo[1,4]oxazine, 3,4-dihydro-2H-benzo[1,4]oxazine, 2,3-dihydro-2H-benzo[1,4]oxazin-4-yl, 3,4-dihydro-2H-benzo[1,4]oxazin-1-yl, 3,4-dihydro-2H-benzo[1,4]oxazin-5-yl, 1H-3H-benzo[de]isochromene, 1H,3H-benzo[de]isochromen-6-yl, 6,7,8,9-tetrahydro-5-oxa-9-benzocycloheptane, 2,3-dihydro-1,4-benzodioxane, 2,3-dihydro-1,4-benzodioxan-5-yl, 7,8-dihydro-6H-5-oxa-9-aza-benzocyclohepten-9-yl, 7,8-dihydro-6H-5-oxa-9-aza-benzocyclohepten-4-yl, benzo-1,4-dioxane, lower alkoxy, amido, alkoxy carbonyl, tetrahydrofuran-2-yl, hydroxyalkoxy, and sulfonamido;

in which

$R^{16}$ and $R^{17}$ are hydrogen or methyl, ethyl, propyl, isopropyl, tert-butyl, butyl, pentyl or n-hexyl; and

$R^{11}$ is hydrogen or methyl, ethyl, propyl, isopropyl, tert-butyl, butyl, pentyl or n-hexyl; provided that:

(i) when $R^9$ is naphthyl, indol-1-yl, or 2,3-dihydroindol-1-yl, and $R^8$, $R^{10}$ and $R^{11}$ are all hydrogen, $R^7$ is not hydrogen, methyl or ethyl;
(ii) when $R^9$ is phenyl or naphthyl, $R^7$ is not $-NR^{12}R^{13}$ ;
(iii) when $R^9$ is phenyl, $R^8$ is not alkoxy with 1 to 6 carbon atoms, and $R^7$ and $R^8$ are not halo;
(iv) when $R^8$ is phenyl and $R^7$ is H, $R^8$ is not hydrogen, methyl or ethyl; and
(v) when $R^9$ is 1,2,3,4-tetrahydroquinolinyl, $R^{10}$ and $R^{11}$ are hydrogen;

or a pharmaceutically acceptable salt or N-oxide thereof.

**9.** The composition or use of claim 8, whereby the serotonin receptor antagonist is 2-amino-4-(4-fluoronaphth-1-yl)-6-isopropyl-pyrimidine.

**10.** The composition or use according to any of claims 1 to 9, whereby the histidine decarboxylase inhibitor is (S)-α-fluoromethyl-histldine.

**11.** The pharmaceutical composition of claims 1 to 10 additionally comprising at least one further pharmaceutically active compound for pain treatment.

**12.** The use of any of claims 2 to 10 wherein the disease state is selected from the diseases migraine, hypertension, disorders of the gastrointestinal tract, restenosis, asthma, obstructive airway disease, bronchial pulmonary dysplasia, prostate hyperplasia, priapism and allergic disorders.

**13.** The use of any of claim 2 to 10 or 12, wherein the disease state comprises pain.

**14.** The use of claim 13 wherein the disease state comprises inflammatory pain, neuropathic pain, cancer pain, acute pain or chronic pain.

**15.** The use of claim 12 wherein the disease state comprises allergic asthma, irritable bowel syndrome, hypertonic lower esophageal sphinter, motility disorders, benign prostate hyperplasia, urticaria or the symptoms of seasonal and perennial allergic rhinitis.

**16.** Use of (S)-α-fluoromethylhistidine for the manufacture of a medicament for the prophylactic treatment of migraine.

European Patent Office

# PARTIAL EUROPEAN SEARCH REPORT

which under Rule 45 of the European Patent Convention shall be considered, for the purposes of subsequent proceedings, as the European search report

Application Number
EP 01 13 0012

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 61159 A (MAX ZELLER SOEHNE AG ;DANZ HENNING (DE); HAMBURGER MATTHIAS) 19 October 2000 (2000-10-19)<br>* page 4, line 15-20; claims 7,10 *<br>* page 4, line 25-27; example 23 *<br>* page 5, line 5-20; table 7 *<br>* page 6, line 10-13 *<br>* page 7, line 32 - page 8, line 5 *<br>--- | 1,2, 11-15 | A61P29/00<br>A61P1/00<br>A61P9/10<br>A61P9/12<br>A61P11/00<br>A61P13/08<br>A61P37/00<br>A61K31/4172<br>A61K45/06<br>A61K31/505<br>A61K31/445<br>A61K31/415 |
| X | BE 752 108 A (MERCK & CO INC) 17 December 1970 (1970-12-17) | 1 | |
| A | * page 2, line 6,7 *<br>* page 2, column 11-14; claim 9 *<br>--- | 2,12,15 | |
| A | WO 95 18132 A (MERCK FROSST CANADA INC) 6 July 1995 (1995-07-06)<br>* page 16-17 *<br>* page 26, line 27,28 *<br>* page 27, line 6-8 *<br>--- | 1,2,10, 12,15 | |
| D,A | GB 2 001 626 A (MERRELL TORAUDE & CO) 7 February 1979 (1979-02-07)<br>* page 1, line 76-114 *<br>* page 3, line 98-127; examples 17,18 *<br>---<br>-/-- | 1-4, 10-15 | TECHNICAL FIELDS SEARCHED (Int.Cl.7)<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 27 June 2002 | KANBIER D.T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 01 13 0012

Claim(s) searched completely:
16

Claim(s) searched incompletely:
1-15

Reason for the limitation of the search:

Present claims 1-7 and 10-15 of the first invention relate to compositions and uses involving compounds and an application defined by reference to desirable characteristics or properties, namely:
(i) antagonism of the serotonin receptor;
(ii) inhibition of histidine decarboxylase;
(iii) ability of a disease state to be alleviated by treatment with a serotonin recptor antagonist and/or histamine receptor antagonist
(iii) inhibition of the serotonin 5-HT2 receptor;
(iv) inhibition of the serotonin 5-HT2b receptor; and
(v) being pharmaceutically active against pain.
The claims cover all compounds having these characteristics or properties, whereas the application provides support within the meaning of Article 84 EPC and/or disclosure within the meaning of Article 83 EPC for only a limited number of such compounds. In the present case, the claims so lack support, and the application so lacks disclosure, that a meaningful search over the whole of the claimed scope is impossible. Independent of the above reasoning, the claims also lack clarity (Article 84 EPC). A compound cannot be sufficiently described by its mechanism of action and/or its pharmacological profile. Again, this lack of clarity in the present case is such as to render a meaningful search over the whole of the claimed scope impossible.

Consequently, the search has been carried out for those parts of the claims which appear to be clear (and concise), supported and disclosed, namely those parts relating to the compounds and therapeutic applications used in the examples and specifically disclosed in the claims, with due regard being given to the description.

European Patent Office

# PARTIAL EUROPEAN SEARCH REPORT

Application Number
EP 01 13 0012

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | EP 0 028 279 A (CALAIRE CHIMIE SA ;LABOREC LAB RECH BIOLOGIQUES)<br>13 May 1981 (1981-05-13)<br>* page 7, line 23-28 *<br>* page 8, column 16-21 *<br>--- | 1 | |
| A | ENGELHARDT, G.: "Pharmacology of 9,10-dihydro-10-(1-methyl-4-piperidinylidene)-9-anthrol (WA 335 BS), a histamine and serotonin antagonist"<br>ARZNEIM.-FORSCH. (1975), 25(11), 1723-37, XP001073991<br>* page 1723-4 *<br>--- | 5-7 | |
| A | LECOMTE, JEAN: "Antiamine activities of a thioxanthene derivative in rats"<br>C. R. SEANCES SOC. BIOL. SES FIL. (1967), 161(5), 1155-8, XP008004959<br>* the whole document *<br>----- | 1 | TECHNICAL FIELDS SEARCHED (Int.Cl.7) |

EPO FORM 1503 03.82 (P04C10)

European Patent Office

Application Number
EP 01 13 0012

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

5-7, partly 1-4, 10-15

European Patent Office

# LACK OF UNITY OF INVENTION SHEET B

Application Number
EP 01 13 0012

The Search Division considers that the present European patent application does not comply with the requirements of unity of invention and relates to several inventions or groups of inventions, namely:

1. Claims: 5-7; partly 1-4, 10-15

   Pharmaceutical combinations of a serotonin receptor antagonist of the general formula of present claim 5 and a histidine decarboxylase inhibitor, for use in treatments of diseases mentioned in claims 12-15.

2. Claims: 8, 9; partly 1-4, 10-15

   Pharmaceutical combinations of a serotonin receptor antagonist of the general formula of present claim 8 and a histidine decarboxylase inhibitor, for use in treatments of diseases mentioned in claims 12-15

3. Claim : 16

   Use of the histidine decarboxylase inhibitor (S)-alpha-fluoromethylhistidine for the manufacture of a medicament for the prophylactic treatment of migraine

## ANNEX TO THE EUROPEAN SEARCH REPORT ON EUROPEAN PATENT APPLICATION NO. EP 01 13 0012

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2002

| Patent document cited in search report | | Publication date | Patent family member(s) | | Publication date |
|---|---|---|---|---|---|
| WO 0061159 | A | 19-10-2000 | EP | 1078634 A1 | 28-02-2001 |
| | | | AU | 3548900 A | 14-11-2000 |
| | | | WO | 0061159 A1 | 19-10-2000 |
| | | | EP | 1171141 A1 | 16-01-2002 |
| BE 752108 | A | 17-12-1970 | BE | 752108 A1 | 17-12-1970 |
| | | | DE | 2029823 A1 | 30-12-1971 |
| | | | DK | 138448 B | 11-09-1978 |
| | | | FI | 54704 B | 31-10-1978 |
| | | | FR | 2052983 A5 | 16-04-1971 |
| | | | GB | 1261660 A | 26-01-1972 |
| | | | IE | 34275 B1 | 19-03-1975 |
| | | | IL | 34687 A | 30-07-1973 |
| | | | MY | 47073 A | 31-12-1973 |
| | | | NL | 7008881 A | 22-12-1970 |
| | | | SE | 397678 B | 14-11-1977 |
| | | | US | 3830827 A | 20-08-1974 |
| | | | NO | 132429 B | 04-08-1975 |
| | | | ZA | 7004123 A | 26-01-1972 |
| WO 9518132 | A | 06-07-1995 | US | 5472964 A | 05-12-1995 |
| | | | AU | 684884 B2 | 08-01-1998 |
| | | | AU | 1378195 A | 17-07-1995 |
| | | | CA | 2180014 A1 | 06-07-1995 |
| | | | WO | 9518132 A1 | 06-07-1995 |
| | | | CN | 1148389 A | 23-04-1997 |
| | | | CZ | 9601879 A3 | 15-01-1997 |
| | | | EP | 0737196 A1 | 16-10-1996 |
| | | | FI | 962639 A | 26-06-1996 |
| | | | HU | 76544 A2 | 29-09-1997 |
| | | | JP | 9507080 T | 15-07-1997 |
| | | | NO | 962717 A | 27-06-1996 |
| | | | SK | 83896 A3 | 05-02-1997 |
| GB 2001626 | A | 07-02-1979 | US | 4315095 A | 09-02-1982 |
| | | | AU | 521354 B2 | 01-04-1982 |
| | | | AU | 3731778 A | 03-01-1980 |
| | | | BE | 869322 A1 | 16-11-1978 |
| | | | CA | 1120041 A1 | 16-03-1982 |
| | | | CH | 638790 A5 | 14-10-1983 |
| | | | DE | 2832309 A1 | 08-02-1979 |
| | | | ES | 471594 A1 | 01-10-1979 |
| | | | FR | 2398736 A1 | 23-02-1979 |
| | | | IE | 46928 B1 | 02-11-1983 |
| | | | IT | 1107454 B | 25-11-1985 |
| | | | JP | 1353020 C | 11-12-1986 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 01 13 0012

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2002

| Patent document cited in search report | | Publication date | Patent family member(s) | | Publication date |
|---|---|---|---|---|---|
| GB 2001626 | A | | JP | 54036262 A | 16-03-1979 |
| | | | JP | 61023792 B | 07-06-1986 |
| | | | NL | 7807871 A | 30-01-1979 |
| | | | NZ | 187537 A | 26-08-1980 |
| | | | US | 4388466 A | 14-06-1983 |
| | | | ZA | 7803350 A | 27-06-1979 |
| EP 0028279 | A | 13-05-1981 | EP | 0028279 A1 | 13-05-1981 |
| | | | AT | 2528 T | 15-03-1983 |
| | | | DE | 2964836 D1 | 24-03-1983 |

EPO FORM P0459

For more details about this annex : see Official Journal of the European Patent Office, No. 12/82